(19) Europäisches Patentamt
European Patent Office
Office européen des brevets

(11) Veröffentlichungsnummer: **0 142 040**
**A2**

(12) # EUROPÄISCHE PATENTANMELDUNG

(21) Anmeldenummer: 84112297.1

(22) Anmeldetag: 12.10.84

(51) Int. Cl.[4]: **C 07 D 239/56**
C 07 D 239/58, C 07 D 239/38
A 01 N 43/54

(30) Priorität: 26.10.83 DE 3338859

(43) Veröffentlichungstag der Anmeldung:
22.05.85 Patentblatt 85/21

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Steinbeck, Karl, Dr.
Rosenkranz 36
D-5093 Burcheid(DE)

(72) Erfinder: Brandes, Wilhelm, Dr.
Eichendorffstrasse 3
D-5653 Leichlingen 1(DE)

(72) Erfinder: Hänssler, Gerd, Dr.
Heymannstrasse 40
D-5090 Leverkusen 1(DE)

(72) Erfinder: Reinecke, Paul, Dr.
Lessingstrasse 11
D-5090 Leverkusen 1(DE)

(54) **Substituierte Pyrimidine.**

(57) Neue substituierte Pyrimidine der allgemeinen Formel (I),

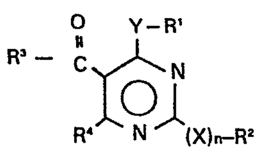

(I)

in welcher
$R^1$, $R^2$, $R^3$, $R^4$, Y, X und n die in der Beschreibung angegebene Bedeutung haben sowie ihre Verwendung als Schädlingsbekämpfungsmittel, vor allem ihre Verwendung als Fungizide oder Bakterizide.

Die neuen substituierten Pyrimidine können nach verschiedenen Verfahren hergestellt werden, indem man z.B. geeignete 4-Halogenpyrimidine mit Alkoholen oder Thiolen umsetzt und gegenbenenfalls die entstehenden Thioverbindungen oxidiert.

EP 0 142 040 A2

Croydon Printing Company Ltd.

BAYER AKTIENGESELLSCHAFT 5090 Leverkusen, Bayerwerk

Konzernverwaltung RP
Patentabteilung

Bas/Hed/c
Typ Ib

## Substituierte Pyrimidine

Die Erfindung betrifft neue substituierte Pyrimidine, mehrere Verfahren zu ihrer Herstellung sowie ihre Verwendung als Schädlingsbekämpfungsmittel.

Bestimmte substituierte Pyrimidine, wie das 5-Ethoxycarbonyl-2-methylthio-4-phenoxy-pyrimidin, das 5-Ethoxycarbonyl-2-methylthio-4-phenylthiopyrimidin und das 5-Ethoxycarbonyl-2-methyl-4-(2-nitrophenylthio)-pyrimidin sind bekannt (vgl. J. Chem. Soc. C, 1967, 1172-1178 sowie J. Chem. Soc. C, 1968, 1753-1761).

Über deren Wirksamkeit als Schädlingsbekämpfungsmittel ist jedoch nichts bekannt.

Weiterhin ist bekannt, daß organische Schwefelverbindungen, wie beispielsweise das Zink-ethylen-1,2-bis-(dithiocarbamat), oder Stickstoffheterocyclen, wie beispielsweise das 1-Triphenylmethylimidazol, fungizide Eigenschaften besitzen (vgl. z.B. US-PS 3 321 366).

Die Wirkung dieser Verbindungen ist jedoch, insbesondere bei niedrigen Aufwandmengen und -konzentrationen, nicht immer in allen Anwendungsbereichen zufriedenstellend.

Es wurden neue substituierte Pyrimidine der allgemeinen Formel (I),

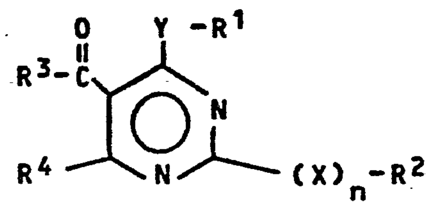

(I)

in welcher

$R^1$ für gegebenenfalls substituiertes Aryl steht,

$R^2$ für Alkyl, für Alkenyl, für gegebenenfalls substituiertes Cycloalkylalkyl oder für gegebenenfalls substituiertes Aralkyl steht,

$R^3$ für Hydroxy, für einen Rest $-O-R^5$ oder für einen Rest $-N\begin{matrix} R^6 \\ R^7 \end{matrix}$ steht,

wobei $R^5$ für Alkyl, für gegebenenfalls substituiertes Cycloalkylalkyl oder für gegebenenfalls substituiertes Aralkyl steht und

0142040

$R^6$ und $R^7$ unabhängig voneinander für Wasserstoff, für Alkyl oder für gegebenenfalls substituiertes Aryl stehen oder

$R^6$ und $R^7$ gemeinsam mit dem Stickstoffatom, an welches sie gebunden sind, für einen gegebenenfalls substituierten gesättigten Heterocyclus stehen, der weitere Heteroatome enthalten kann,

$R^4$ für Wasserstoff oder Alkyl steht,

X für Schwefel oder die Sulfonylgruppe steht,

Y für Sauerstoff oder Schwefel steht,

n für 0 oder 1 steht,

wobei $R^1$ nur dann für unsubstituiertes Phenyl steht, wenn nicht gleichzeitig $R^2$ für Methyl, n für 1 und X für Schwefel stehen und wobei $R^1$ nur dann für 2-Nitrophenyl steht, wenn nicht gleichzeitig Y für Schwefel, n für 0 und $R^2$ für Methyl stehen, sowie deren pflanzenverträgliche Säureadditionssalze gefunden.

Weiterhin wurde gefunden, daß man die neuen substituierten Pyrimidine der allgemeinen Formel (I),

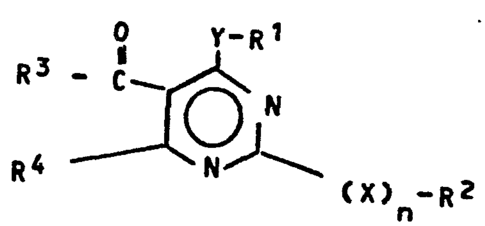

(I)

in welcher

$R^1$ für gegebenenfalls substituiertes Aryl steht,

$R^2$ für Alkyl, für Alkenyl, für gegebenenfalls substituiertes Cycloalkylalkyl oder für gegebenenfalls substituiertes Aralkyl steht,

$R^3$ für Hydroxy, für einen Rest $-O-R^5$ oder für einen

Rest 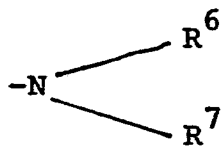 steht,

wobei $R^5$ für Alkyl, für gegebenenfalls substituiertes Cycloalkylalkyl oder für gegebenenfalls substituiertes Aralkyl steht und

$R^6$ und $R^7$ unabhängig voneinander für Wasserstoff, für Alkyl oder für gegebenenfalls substituiertes Aryl stehen oder

$R^6$ und $R^7$ gemeinsam mit dem Stickstoffatom, an welches sie gebunden sind, für einen gegebenenfalls substituierten gesättigten Heterocyclus stehen, der weitere Heteroatome enthalten kann,

$R^4$ für Wasserstoff oder Alkyl steht,

X für Schwefel oder die Sulfonylgruppe steht,

Y für Sauerstoff oder Schwefel steht,

n für 0 oder 1 steht,

wobei $R^1$ nur dann für unsubstituiertes Phenyl steht, wenn nicht gleichzeitig $R^2$ für Methyl, n für 1 und X für Schwefel stehen, und wobei $R^1$ nur dann für 2-Nitrophenyl steht, wenn nicht gleichzeitig Y für Schwefel, n für 0 und $R^2$ für Methyl stehen, sowie deren pflanzenverträglichen Säureadditionssalze erhält, wenn man

(a) 4-Halogenpyrimidine der Formel (II),

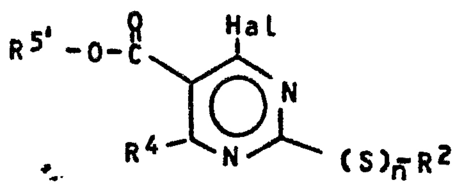 (II)

in welcher

Hal für Halogen steht,

$R^{5'}$ für Alkyl steht und

$R^2$,$R^4$ und n die oben angegebene Bedeutung haben,

mit aromatischen Alkoholen oder Thiolen der Formel (III)

$$R^1 - Y - H \qquad (III)$$

in welcher

$R^1$ und Y die oben angegebene Bedeutung haben,

gegebenenfalls in Gegenwart eines Verdünnungsmittels und gegebenenfalls in Gegenwart eines Säurebindemittels umsetzt zu den Verbindungen der Formel (Ia)

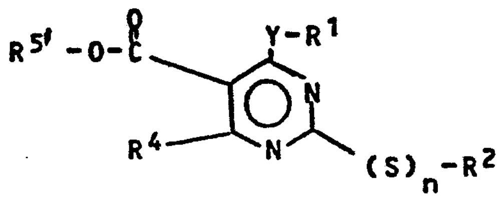

(Ia)

in welcher

$R^1$, $R^2$, $R^4$, $R^{5'}$, Y und n die oben angegebene Bedeutung haben,

oder wenn man

(b) die nach Verfahren (a) erhaltenen 5-Alkoxycarbonyl-pyrimidine der Formel (Ia)

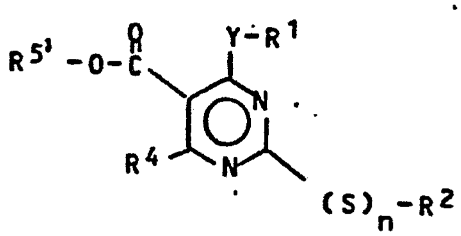

(Ia)

in welcher

$R^1$,$R^2$,$R^4$,$R^{5'}$,Y und n die oben angegebene Bedeutung haben,

mit Basen gegebenenfalls in Gegenwart eines Verdünnungsmittels in üblicher Weise verseift zu den Pyrimidin-5-carbonsäuren der Formel (Ib)

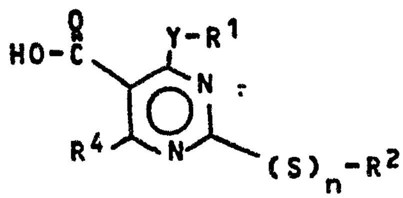

(Ib)

in welcher

$R^1$,$R^2$,$R^4$,Y und n die oben angegebene Bedeutung haben,

oder wenn man

(c) die nach Verfahren (b) erhältlichen Pyrimidin-5-carbonsäuren der Formel (Ib),

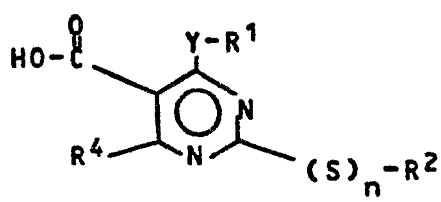

(Ib)

in welcher

$R^1$,$R^2$,$R^4$,Y und n die oben angegebene Bedeutung haben,

α) mit Alkoholen der Formel (IV)

$$R^5 - OH \qquad (IV)$$

in welcher

$R^5$ die oben angegebene Bedeutung hat,

gegebenenfalls in Gegenwart eines Verdünnungsmittels, und gegebenenfalls in Gegenwart eines Katalysators oder Aktivierungsmittels sowie gegebenenfalls in Gegenwart eines Säurebindemittels umsetzt zu den Pyrimidin-5-carbonsäureestern der Formel (Ic)

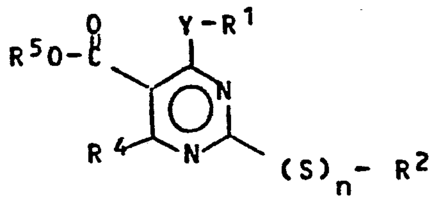

(Ic)

in welcher

$R^1$,$R^2$,$R^4$,$R^5$,Y und n die oben angegebene Bedeutung haben,

oder

(β) mit Aminen der Formel (V),

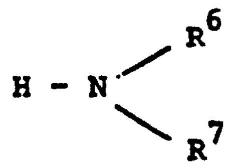 (V)

in welcher

$R^6$ und $R^7$ die oben angegebene Bedeutung haben,

gegebenenfalls in Gegenwart eines Verdünnungsmittels, und gegebenenfalls in Gegenwart eines Aktivierungsmittels sowie gegebenenfalls in Gegenwart eines Säurebindemittels umsetzt zu den Pyrimidin-5-carbonsäureamiden der Formel (Id)

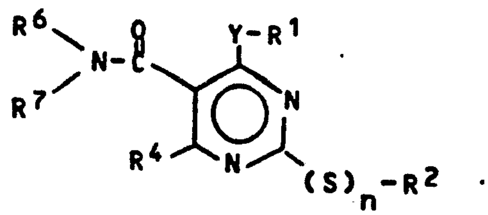 (Id)

in welcher

$R^1$,$R^2$,$R^4$,$R^6$,$R^7$,Y und n die oben angegebene Bedeutung haben,

oder wenn man

(d) die nach Verfahren (a), (b), (c-α) oder (c-ß) erhältlichen Pyrimidin-Derivate der Formel

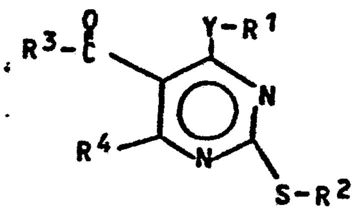

(Ie)

in welcher

$R^1$, $R^2$, $R^3$, $R^4$ und Y die oben angegebene Bedeutung haben,

mit Oxidationsmitteln der Formel (VI)

R - O - OH (VI)

in welcher

R für Wasserstoff oder für jeweils gegebenenfalls substituiertes Alkanoyl oder Aroyl steht,

gegebenenfalls in Gegenwart eines Verdünnungsmittels, und gegebenenfalls in Gegenwart eines Katalysators sowie gegebenenfalls in Gegenwart eines Säurebindemittels oxidiert zu den 2-Sulfonylpyrimidinen der Formel (If)

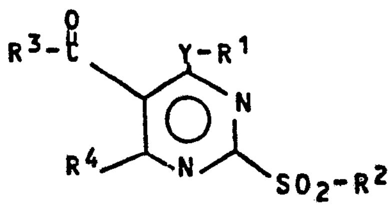

(If)

in welcher

$R^1$, $R^2$, $R^3$, $R^4$ und Y die oben angegebene Bedeutung haben,

und gegebenenfalls anschließend an die nach Verfahren (a), (b), (c-α), (c-β) oder (d) erhaltenen Pyrimidin-Derivate der Formel (I) eine Säure addiert.

Schließlich wurde gefunden, daß die neuen substituierten Pyrimidine der allgemeinen Formel (I) fungizide und bakterizide Eigenschaften besitzen. Überraschenderweise zeigen die neuen substituierten Pyrimidine der allgemeinen Formel (I) eine bessere fungizide und bakterizide Wirksamkeit als die aus dem Stand der Technik bekannten Verbindungen Zink-ethylen-1,2-bis-(dithiocarbamat) oder 1-Triphenylmethylimidazol, welches wirkungsmäßig naheliegende Verbindungen sind.

Die neuen substituierten Pyrimidine der Formel (I) stellen somit einen Fortschritt der Technik dar.

Die substituierten Pyrimidine sind durch die Formel (I) allgemein definiert. Bevorzugt sind Verbindungen der Formel (I), bei welchen

$R^1$ für gegebenenfalls einfach oder mehrfach, gleich oder verschieden substituiertes Aryl mit 6 bis 10 Kohlenstoffatomen steht, wobei als Substituenten infrage kommen: Halogen, jeweils geradkettiges oder verzweigtes, gegebenenfalls einfach oder mehrfach, gleich oder verschieden durch Halogen substituiertes Alkyl, Alkoxy oder Alkylthio mit jeweils 1 bis 12 Kohlenstoffatomen, gegebenenfalls einfach oder mehrfach, gleich oder verschieden durch geradkettiges oder verzweigtes Alkyl mit 1 bis 4 Kohlenstoffatomen substituiertes zweifach verknüpftes Alkylen mit 3 bis 5 Kohlenstoffatomen, gegebenenfalls einfach oder mehrfach, gleich oder verschieden durch Halogen oder geradkettiges oder verzweigtes Alkyl mit 1 bis 4 Kohlenstoffatomen substituiertes Cycloalkyl mit 3 bis 7 Kohlenstoffatomen, Amino, N-Alkylamino oder N,N-Dialkylamino mit jeweils 1 bis 4 Kohlenstoffatomen in den einzelnen geradkettigen oder verzweigten Alkylteilen, Alkoxycarbonyl mit 1 bis 10 Kohlenstoffatomen im geradkettigen oder verzweigten Alkylteil oder Phenyl,

$R^2$ für jeweils geradkettiges oder verzweigtes Alkyl oder Alkenyl mit jeweils bis zu 10 Kohlenstoffatomen, für gegebenenfalls einfach oder mehrfach, gleich oder verschieden durch Halogen, geradkettiges oder verzweigtes Alkyl mit 1 bis 4 Kohlenstoffatomen im Cycloalkylteil substituiertes

Cycloalkylalkyl mit 1 bis 4 Kohlenstoffatomen im geradkettigen oder verzweigten Alkylteil und 3 bis 7 Kohlenstoffatomen im Cycloalkylteil oder für gegebenenfalls einfach oder mehrfach, gleich oder verschieden durch Halogen oder jeweils geradkettiges oder verzweigtes Alkyl oder Alkoxy mit jeweils 1 bis 4 Kohlenstoffatomen im Arylteil substituiertes Aralkyl mit 1 bis 4 Kohlenstoffatomen im geradkettigen oder verzweigten Alkylteil und 6 bis 10 Kohlenstoffatomen im Arylteil steht,

$R^3$ für Hydroxy, für einen Rest $-O-R^5$ oder für einen Rest 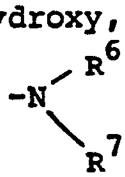 steht, wobei

$R^5$ für geradkettiges oder verzweigtes Alkyl mit 1 bis 10 Kohlenstoffatomen oder für gegebenenfalls einfach oder mehrfach, gleich oder verschieden durch Halogen, geradkettiges oder verzweigtes Alkyl mit 1 bis 4 Kohlenstoffatomen im Cycloalkylteil substituiertes Cycloalkylalkyl mit 1 bis 4 Kohlenstoffatomen im geradkettigen oder verzweigten Alkylteil und 3 bis 7 Kohlenstoffatomen im Cycloalkylteil oder für gegebenenfalls einfach oder mehrfach, gleich oder verschieden durch Halogen oder jeweils geradkettiges oder verzweigtes Alkyl oder Alkoxy mit jeweils 1 bis 4 Kohlenstoffatomen im Arylteil substituiertes Aralkyl mit 1 bis 4 Kohlenstoffatomen im geradkettigen oder verzweigten Alkylteil und 6 bis 10 Kohlenstoffatomen im Arylteil steht, und

$R^6$ und $R^7$ unabhängig voneinander jeweils für Wasserstoff, für geradkettiges oder verzweigtes Alkyl mit 1 bis 10 Kohlenstoffatomen oder für gegebenenfalls einfach oder mehrfach, gleich oder verschieden durch Halogen oder jeweils geradkettiges oder verzweigtes Alkyl oder Alkoxy mit jeweils 1 bis 4 Kohlenstoffatomen substituiertes Aryl mit 6 bis 10 Kohlenstoffatomen stehen oder

$R^6$ und $R^7$ gemeinsam mit dem Stickstoffatom, an welches sie gebunden sind, für einen gegebenenfalls einfach oder mehrfach, gleich oder verschieden durch Alkyl mit 1 bis 4 Kohlenstoffatomen substituierten gesättigten 5- bis 7-gliedrigen Heterocyclus stehen, der ein Stickstoff- oder Sauerstoffatom als weiteres Heteroatom enthalten kann,

$R^4$ für Wasserstoff oder für geradkettiges oder verzweigtes Alkyl mit 1 bis 8 Kohlenstoffatomen steht,

X für Schwefel oder die Sulfonylgruppe steht,

Y für Sauerstoff oder Schwefel steht und

n für 0 oder 1 steht,

wobei $R^1$ nur dann für unsubstituiertes Phenyl steht, wenn nicht gleichzeitig $R^2$ für Methyl, n für 1 und X für Schwefel stehen und wobei $R^1$ nur dann für 2-Nitrophenyl steht, wenn nicht gleichzeitig Y für Schwefel, n für 0 und $R^2$ für Methyl stehen.

Besonders bevorzugt sind Verbindungen der allgemeinen Formel (I), bei welchen

$R^1$ für gegebenenfalls ein- bis fünffach, gleich oder verschieden substituiertes Phenyl oder Naphthyl steht, wobei als Substituenten insbesondere infrage kommen: Fluor, Chlor, Brom, Methyl, Ethyl, jeweils geradkettiges oder verzweigtes Propyl, Butyl, Pentyl, Hexyl, Heptyl, Octyl, Nonyl, Decyl oder Dodecyl, Methoxy, Ethoxy, jeweils geradkettiges oder verzweigtes Propyloxy, Butyloxy, Pentyloxy oder Hexyloxy, Methylthio, Ethylthio, jeweils geradkettiges oder verzweigtes Propylthio, Butylthio, Pentylthio oder Hexylthio, Trifluormethyl, Trichlormethyl, Trifluormethoxy, Trifluormethylthio, Trichlormethoxy, Trichlormethylthio, Propandiyl, Trimethylpropandiyl, Tetramethylpropandiyl, Dimethylpropandiyl, Triethylpropandiyl, Tetraethylpropandiyl, Butandiyl, Trimethylbutandiyl, Tetramethylbutandiyl, Tetraethylbutandiyl, jeweils gegebenenfalls ein- bis dreifach, gleich oder verschieden durch Chlor, Brom, Methyl oder Ethyl substituiertes Cyclopropyl, Cyclopentyl, Cyclohexyl, Cycloheptyl, Methoxycarbonyl, Ethoxycarbonyl, n- und i-Propoxycarbonyl, n- und i-Butoxycarbonyl, n- und i-Pentoxycarbonyl, n- und i-Hexyloxycarbonyl, Amino, N-Methylamino, N,N-Dimethylamino, N-Ethylamino, N,N-Diethylamino, N,N-Dipropylamino, N,N-Dibutylamino oder Phenyl,

$R^2$ für Methyl, Ethyl, n- und i-Propyl, n- und i-Butyl, n- und i-Pentyl, n- und i-Hexyl, n- und i-Heptyl, n- und i-Octyl, n- und i-Decyl, n- und i-Dodecyl, für Allyl, Butenyl, Pentenyl, Hexenyl, für jeweils gegebenenfalls ein- bis fünffach, gleich oder verschieden durch Fluor, Chlor, Brom, Methyl oder Ethyl substituiertes Cyclopropylmethyl oder Cyclohexylmethyl, oder für jeweils gegebenenfalls ein- bis dreifach, gleich oder verschieden durch Fluor, Chlor, Brom, Methyl, Methoxy, Ethyl, Ethoxy, n- und i-Propyl sowie n- und i-Propoxy substituiertes Benzyl oder Phenylethyl steht,

$R^3$ für Hydroxy, Methoxy, Ethoxy, n- und i-Propoxy, n- und i-Butoxy, n- und i-Pentoxy, n- und i-Hexyloxy, n- und i-Octyloxy, n- und i-Decyloxy, für jeweils gegebenenfalls ein- bis fünffach, gleich oder verschieden durch Fluor, Chlor, Brom, Methyl oder Ethyl substituiertes Cyclopropylmethoxy oder Cyclohexylmethoxy, für jeweils gegebenenfalls ein- bis dreifach, gleich oder verschieden durch Fluor, Chlor, Brom, Methyl, Methoxy, Ethyl, Ethoxy,

n- und i-Propyl, sowie n- und i-Propoxy substituiertes Benzyl oder Phenylethyl, für Amino, N-Methylamino, N-Ethylamino, N-Propylamino, N-Butylamino, N-Pentylamino, N-Hexylamino, N-Octylamino, N,N-Dimethylamino, N,N-Diethylamino, N,N-Dipropylamino, N,N-Dibutylamino, N,N-Dihexylamino, für gegebenenfalls ein- bis dreifach, gleich oder verschieden durch Fluor, Chlor, Brom, Methyl, Methoxy, Ethyl oder Ethoxy substituiertes N-Phenylamino oder für jeweils gegebenenfalls ein- bis dreifach, gleich oder verschieden durch Methyl, Ethyl und Isopropyl substituiertes 1-Piperidinyl, 1-Pyrrolidinyl, 4-Morpholinyl, 1-Perhydroazepinyl oder 1-Piperazinyl steht,

$R^4$ für Wasserstoff, Methyl, Ethyl, n- und i-Propyl, sowie n- und i-Butyl steht,

X für Schwefel oder die Sulfonylgruppe steht,

Y für Sauerstoff oder Schwefel steht und

n für 0 oder 1 steht,

wobei $R^1$ nur dann für unsubstituiertes Phenyl steht, wenn nicht gleichzeitig $R^2$ für Methyl, n für 1 und X für Schwefel stehen und wobei $R^1$ nur dann für 2-Nitrophenyl steht, wenn nicht gleichzeitig Y für Schwefel, n für 0 und $R^2$ für Methyl stehen.

Im einzelnen seien außer den bei den Herstellungsbeispielen genannten Verbindungen die folgenden Verbindungen der allgemeinen Formel (I) genannt:

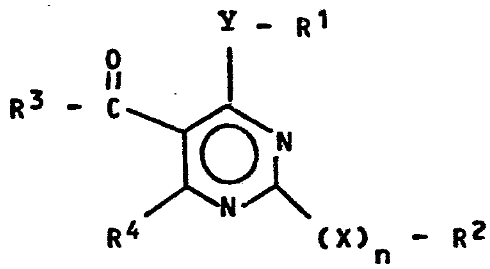

(I)

| $R^1$ | $R^2$ | $R^3$ | $R^4$ | X | Y | n |
|---|---|---|---|---|---|---|
| 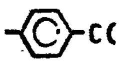$-C(CH_3)_3$ | $-CH_2-CH(CH_3)_2$ | $-OCH_3$ | H | S | O | 1 |
| 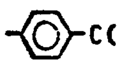$-C(CH_3)_3$ | $-CH_2-CH(CH_3)_2$ | $-OC_3H_7-n$ | H | S | O | 1 |
| 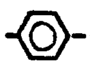$-C(CH_3)_3$ | $-CH_2-CH(CH_3)_2$ | $-OC_3H_7-i$ | H | S | O | 1 |
| 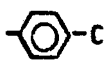$-C(CH_3)_3$ | $-CH_2-CH(CH_3)_2$ | $-OC_6H_{13}-n$ | H | S | O | 1 |
| 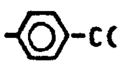$-C(CH_3)_3$ | $-CH_2-CH(CH_3)_2$ | $-OCH_2-$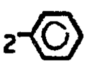 | H | S | O | 1 |
| 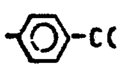$-C(CH_3)_3$ | $-CH_2-CH(CH_3)_2$ | $-O-CH_2$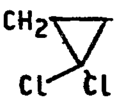 | H | S | O | 1 |
| 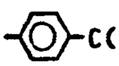$-C(CH_3)_3$ | $-CH_2-CH(CH_3)_2$ | $-OC_2H_5$ | $CH_3$ | S | O | 1 |
| 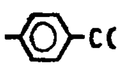$-C(CH_3)_3$ | $-CH(CH_3)_2$ | $-OC_2H_5$ | H | - | O | 0 |
| 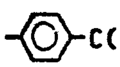$-C(CH_3)_3$ | $-CH_2$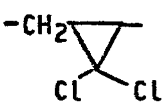 | $-OC_2H_5$ | H | S | O | 1 |
| 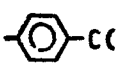$-C(CH_3)_3$ | $-CH_2$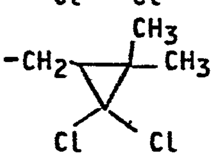 | $-OC_2H_5$ | H | S | O | 1 |
| 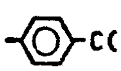$-C(CH_3)_3$ | $-C_6H_{13}-n$ | $-OC_2H_5$ | H | S | O | 1 |

| $R^1$ | $R^2$ | $R^3$ | $R^4$ | X | Y | n |
|---|---|---|---|---|---|---|
| 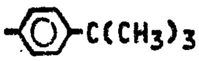 | 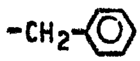 | $-OC_2H_5$ | H | S | O | 1 |
| 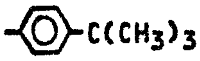 | $-CH_2-CH=CH_2$ | $-OC_2H_5$ | H | S | O | 1 |
| 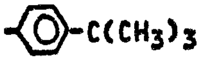 | $-CH_2-CH(CH_3)_2$ | $-OC_2H_5$ | H | $SO_2$ | O | 1 |
| 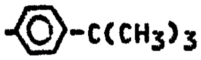 | $-CH_2-CH(CH_3)_2$ | $-OC_2H_5$ | H | S | S | 1 |
| 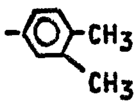 | $-CH_2-CH(CH_3)_2$ | $-OC_2H_5$ | H | S | O | 1 |
| 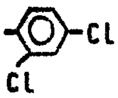 | $-CH_2-CH(CH_3)_2$ | $-OC_2H_5$ | H | S | O | 1 |
| 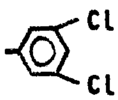 | $-CH_2-CH(CH_3)_2$ | $-OC_2H_5$ | H | S | O | 1 |
| 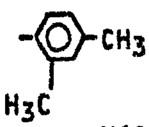 | $-CH_2-CH(CH_3)_2$ | $-OC_2H_5$ | H | S | O | 1 |
| 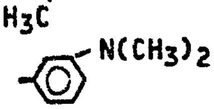 | $-CH_2-CH(CH_3)_2$ | $-OC_2H_5$ | H | S | O | 1 |
| 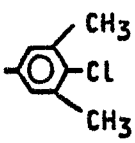 | $-CH_2-CH(CH_3)_2$ | $-OC_2H_5$ | H | S | O | 1 |
| 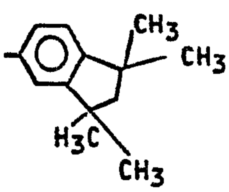 | $-CH_2-CH(CH_3)_2$ | $-OC_2H_5$ | H | S | O | 1 |
| 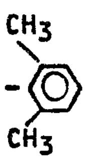 | $-CH_2-CH(CH_3)_2$ | $-OC_2H_5$ | H | S | O | 1 |
| 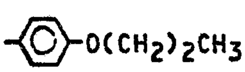 | $-CH_2-CH(CH_3)_2$ | $-OC_2H_5$ | H | S | O | 1 |
| 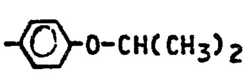 | $-CH_2-CH(CH_3)_2$ | $-OC_2H_5$ | H | S | O | 1 |
| 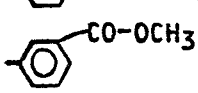 | $-CH_2-CH(CH_3)_2$ | $-OC_2H_5$ | H | S | O | 1 |

| $R^1$ | $R^2$ | $R^3$ | $R^4$ | X | Y | n |
|---|---|---|---|---|---|---|
| 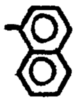 | $-CH_2-CH(CH_3)_2$ | $-OC_2H_5$ | H | S | O | 1 |
| 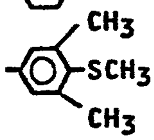 | $-CH_2-CH(CH_3)_2$ | $-OC_2H_5$ | H | S | O | 1 |
| 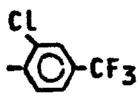 | $-CH_2-CH(CH_3)_2$ | $-OC_2H_5$ | H | S | O | 1 |
| 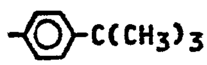 | $-CH_2-CH(CH_3)_2$ | $-OC_2H_5$ | H | S | S | 1 |
| 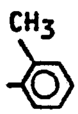 | $-CH(CH_3)_2$ | $-OC_2H_5$ | H | S | S | 1 |
| 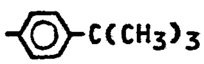 | $-CH(CH_3)_2$ | $-OC_2H_5$ | $CH_3$ | S | S | 1 |
| 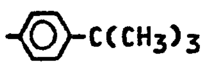 | $-CH(CH_3)_2$ | $-OC_2H_5$ | $CH_3$ | S | O | 1 |
| 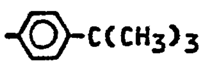 | $-CH(CH_3)_2$ | $-OC_2H_5$ | $CH_3$ | - | O | 0 |
| 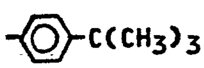 | $-CH(CH_3)_2$ | $-OC_2H_5$ | $CH_3$ | - | S | 0 |
| 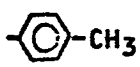 | $-CH(CH_3)_2$ | $-OC_2H_5$ | $CH_3$ | - | S | 0 |
| 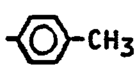 | $-CH(CH_3)_2$ | $-OC_2H_5$ | $CH_3$ | - | O | 0 |
| 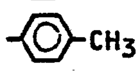 | $-CH(CH_3)_2$ | $-OC_2H_5$ | H | - | S | 0 |
| 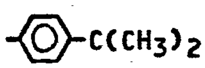 | $-C_2H_5$ | $-OC_2H_5$ | H | S | S | 1 |
| 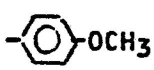 | $-CH_2-CH(CH_3)_2$ | $-OC_2H_5$ | H | S | O | 1 |
| 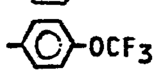 | $-CH_2-CH(CH_3)_2$ | $-OC_2H_5$ | H | S | O | 1 |
| 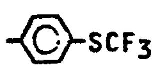 | $-CH_2-CH(CH_3)_2$ | $-OC_2H_5$ | H | S | O | 1 |
| 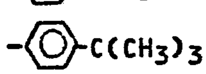 | $-CH_2-CH(CH_3)_2$ | $-NH-CH_3$ | H | S | O | 1 |
| 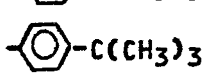 | $-CH(CH_3)_2$ | $-NH-CH_3$ | H | S | O | 1 |
| 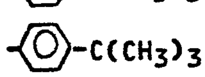 | $-CH_2-CH(CH_3)_2$ | $-N(CH_3)_2$ | H | S | O | 1 |
| 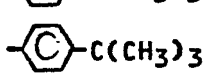 | $-CH_2-CH(CH_3)_2$ | $-NH-C_2H_5$ | H | S | O | 1 |
| 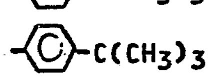 | $-CH_2-CH(CH_3)_2$ | $-N(C_2H_5)_2$ | H | S | O | 1 |

| $R^1$ | $R^2$ | $R^3$ | $R^4$ | X | Y | n |
|---|---|---|---|---|---|---|
| 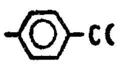$-C(CH_3)_3$ | $-CH_2-CH(CH_3)_2$ | $-N(C_2H_5)_2$ | H | S | O | 1 |
| 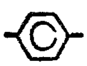$-C(CH_3)_3$ | $-CH_2-CH(CH_3)_2$ | 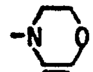 | H | S | O | 1 |
| 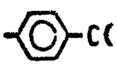$-C(CH_3)_3$ | $-CH_2-CH(CH_3)_2$ | 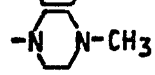 | H | S | O | 1 |
| 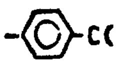$-C(CH_3)_3$ | $-CH_2-CH(CH_3)_2$ | 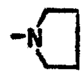 | H | S | O | 1 |
| 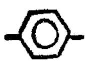$-C(CH_3)_3$ | $-CH_2-CH(CH_3)_2$ | 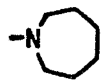 | H | S | O | 1 |
| 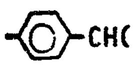$-CH(CH_3)_2$ | $-CH_2-CH(CH_3)_2$ | 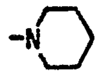 | H | S | O | 1 |
| 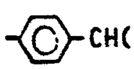$-CH(CH_3)_2$ | $-CH_2-CH(CH_3)_2$ | $-N(C_2H_5)_2$ | H | S | O | 1 |
| 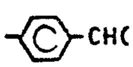$-CH(CH_3)_2$ | $-CH_2-CH(CH_3)_2$ | 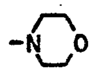 | H | S | O | 1 |
| 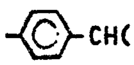$-CH(CH_3)_2$ | $-CH_2-CH(CH_3)_2$ | 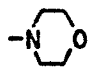 | $CH_3$ | S | O | 1 |
| 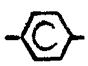$-CH_3$ | $-CH_2-CH(CH_3)_2$ | 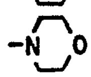 | $CH_3$ | S | O | 1 |
| 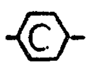$-OCH_3$ | $-CH_2-CH(CH_3)_2$ | 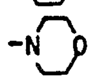 | $CH_3$ | S | O | 1 |
| 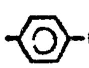$-OCH_3$ | $-CH_2-CH(CH_3)_2$ | 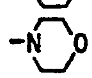 | $C_2H_5$ | S | O | 1 |
| 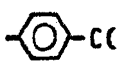$-C(CH_3)_3$ | $-CH_2-CH(CH_3)_2$ | $-OC_2H_5$ | $-C_3H_7-n$ | S | O | 1 |
| 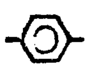$-C(CH_3)_3$ | $-CH_2-CH(CH_3)_2$ | $-OC_2H_5$ | $-C_3H_7-i$ | S | O | 1 |
| 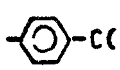$-C(CH_3)_3$ | $-CH_2-CH(CH_3)_2$ | $-OC_2H_5$ | $-C_3H_7-i$ | - | O | 0 |
| 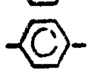$-C(CH_3)_3$ | $-CH_2-CH(CH_3)_2$ | $-OCH_3$ | $CH_3$ | - | O | 0 |
| 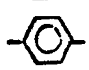$-CH(CH_3)_2$ | $-CH(CH_3)_2$ | $-OCH_3$ | $CH_3$ | - | S | 0 |
| 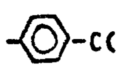$-C(CH_3)_3$ | $-CH(CH_3)_2$ | $-OCH_3$ | $CH_3$ | - | O | 0 |
| 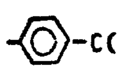$-C(CH_3)_3$ | $-CH(CH_3)_2$ | $-OCH_3$ | $CH_3$ | - | S | 0 |
| 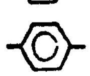$-C(CH_3)_3$ | $-CH(CH_3)_2$ | $-OCH_3$ | $-C_3H_7-i$ | - | O | 0 |
| 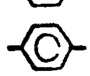$-C(CH_3)_3$ | $-CH(CH_3)_2$ | $-OCH_3$ | $-C_3H_7-i$ | - | S | 0 |
| 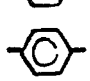$-C(CH_3)_3$ | $-CH(CH_3)_2$ | $-OCH_3$ | $-C_3H_7-i$ | S | S | 1 |
| 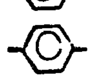$-C(CH_3)_3$ | $-CH(CH_3)_2$ | $-OCH_3$ | $-C_3H_7-i$ | S | O | 1 |
| 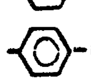$-CH_3$ | $-CH(CH_3)_2$ | $-OCH_3$ | $-C_3H_7-i$ | S | O | 1 |

| $R^1$ | $R^2$ | $R^3$ | $R^4$ | X | Y | n |
|---|---|---|---|---|---|---|
| 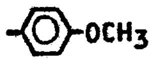 | $-CH(CH_3)_2$ | $-OCH_3$ | $-C_3H_7$-i | S | O | 1 |
| 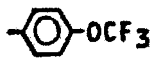 | $-CH(CH_3)_2$ | $-OCH_3$ | $-C_3H_7$-i | S | O | 1 |
| 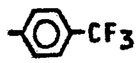 | $-CH(CH_3)_2$ | $-OCH_3$ | $-C_3H_7$-i | S | O | 1 |
| 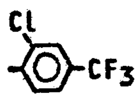 | $-CH(CH_3)_2$ | $-OCH_3$ | $C_3H_7$-i | S | O | 1 |
| 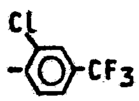 | $-CH_2-CH=CH_2$ | $-OCH_3$ | H | S | O | 1 |
| 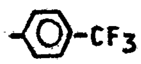 | $-CH_2-CH=CH_2$ | $-OCH_3$ | H | S | O | 1 |

Verwendet man als Ausgangsstoffe beispielsweise 4-Chlor-5-ethoxycarbonyl-2-ethylthiopyrimidin und 3,5-Dimethylphenol, so läßt sich der Reaktionsablauf des erfindungsgemäßen Verfahrens (a) durch das folgende Formelschema darstellen:

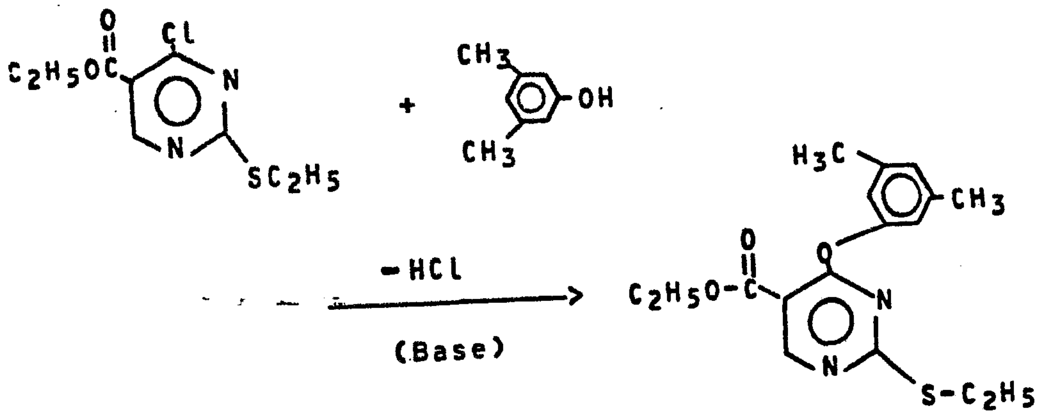


Verwendet man als Ausgangsstoff beispielsweise 4-(3,5-Dimethylphenoxy)-5-ethoxycarbonyl-2-methylthiopyrimidin, so läßt sich der Reaktionsablauf des erfindungsgemäßen Verfahrens (b) durch das folgende Formelschema darstellen:

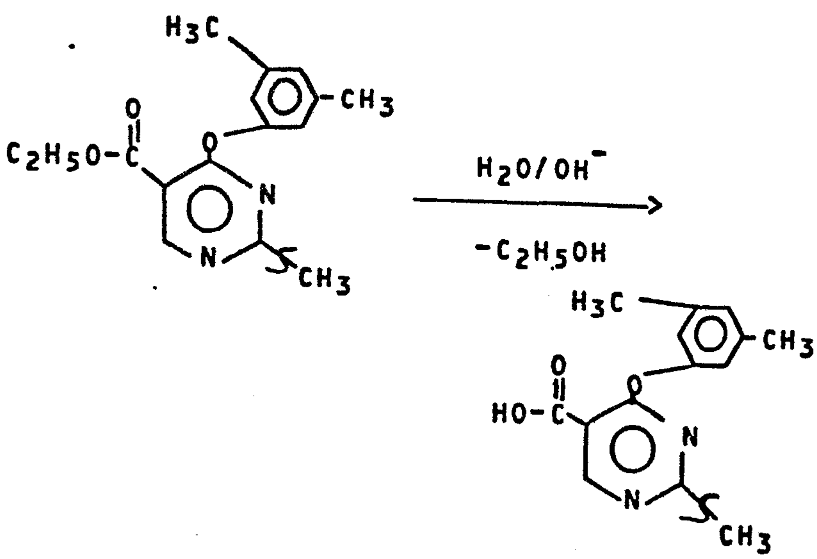

Verwendet man als Ausgangsstoffe beispielsweise 2-Isobutylthio-4-(4-t-butylphenoxy)-pyrimidin-5-carbonsäure und Benzylalkohol sowie p-Toluolsulfonsäure als Katalysator, so läßt sich der Reaktionsablauf des erfindungsgemäßen Verfahrens (c- α ) durch das folgende Formelschema darstellen:

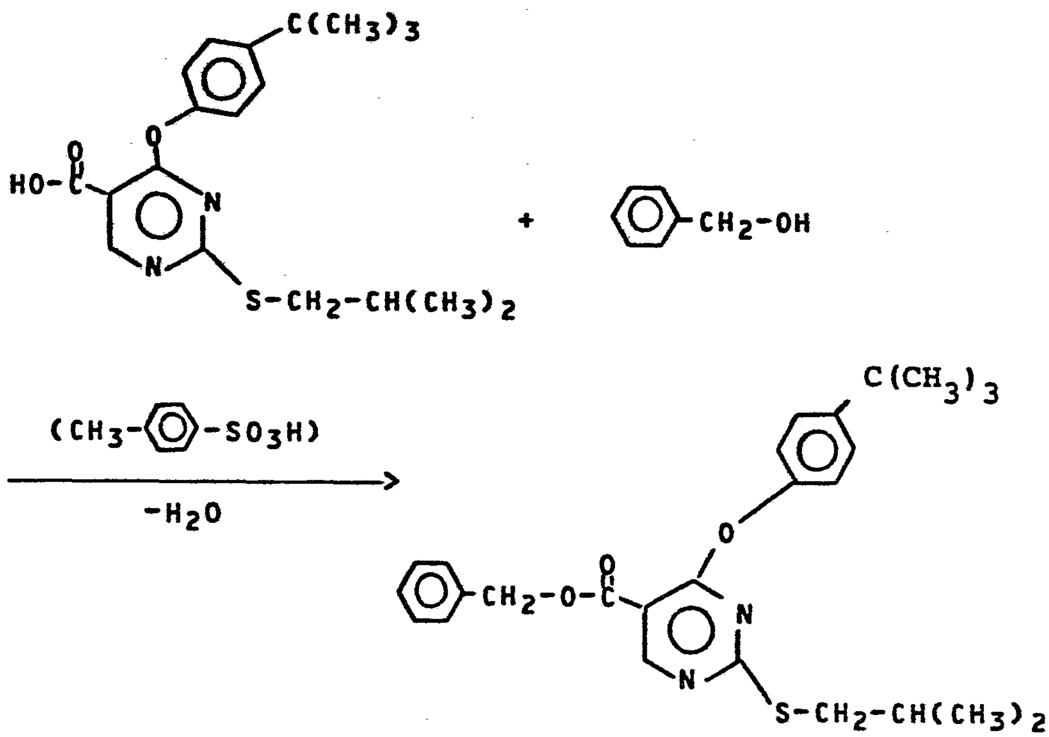


Verwendet man als Ausgangsstoffe beispielsweise 2-Ethylthio-4-(4-t-butylphenoxy)-pyrimidin-5-carbonsäure und Piperidin sowie Thionylchlorid als Aktivierungsmittel, so läßt sich der Reakionsablauf des erfindungsgemäßen Verfahrens (c-ß) durch das folgende Formelschema wiedergeben:

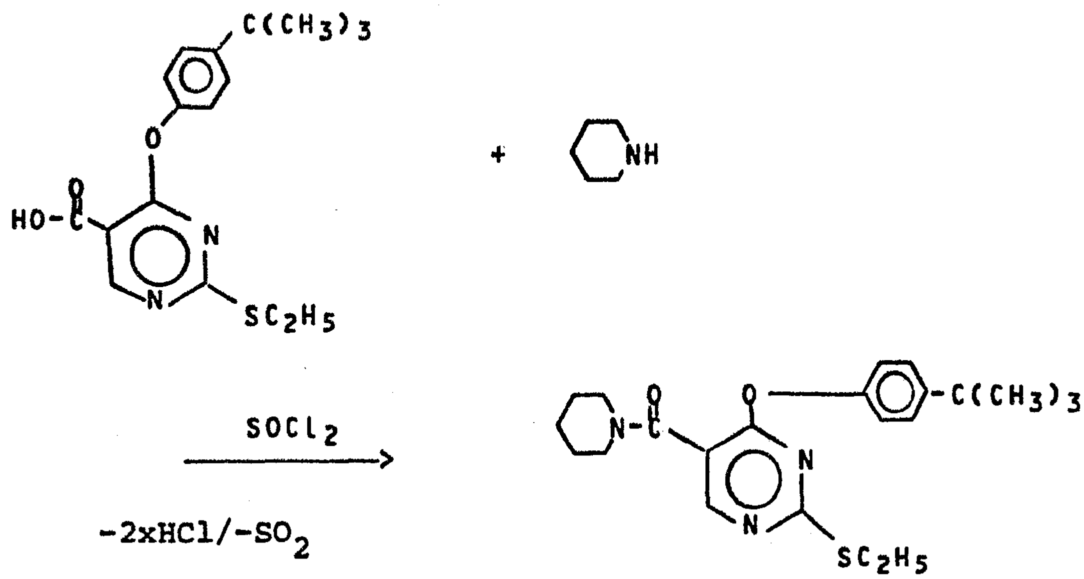


Verwendet man als Ausgangsstoffe beispielsweise 5-Ethoxycarbonyl-2-isobutylthio-4-(4-t-butylphenoxy)-pyrimidin und m-Chlorperbenzoesäure, so läßt sich der Reaktionsablauf des erfindungsgemäßen Verfahrens (d) durch das folgende Formelschema darstellen:

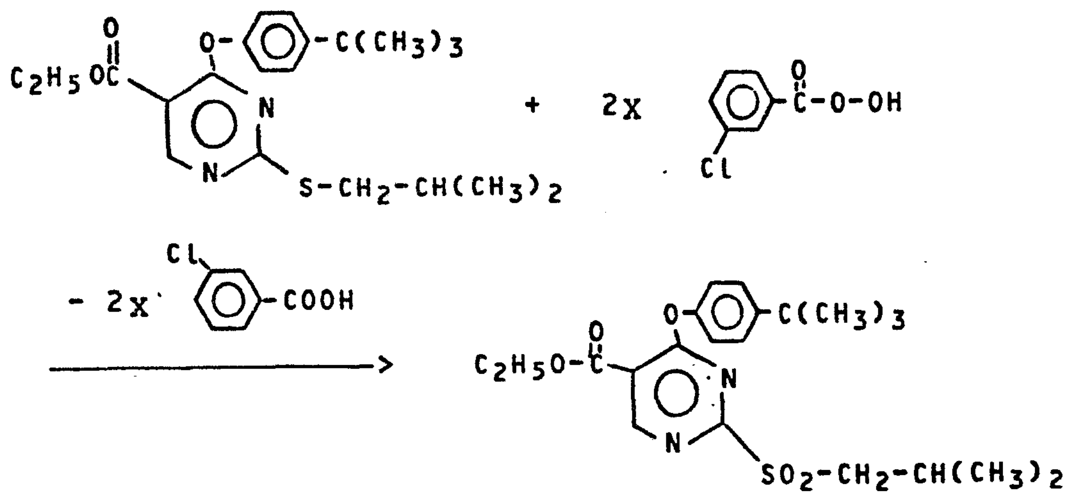

Die zur Durchführung des erfindungsgemäßen Verfahrens (a) als Ausgangsstoffe benötigten 4-Halogenpyrimidine sind durch die Formel (II) allgemein definiert. In dieser Formel (II) stehen $R^2$, $R^4$ und n für diejenigen Reste, die schon bei der Beschreibung der erfindungsgemäßen Stoffe der Formel (I) für diese Reste angegeben wurden. $R^{5'}$ steht vorzugsweise für Methyl oder Ethyl, Hal steht vorzugsweise für Chlor oder Brom.

Die 4-Halogenpyrimidine der Formel (II) sind bekannt (vgl. z.B. US 4 118 217 oder D.J. Brown "The Pyrimidines", Interscience Publishers, J. Wiley and Sons., New York, London, 1962, S. 39, S. 162) oder lassen sich nach bekannten Methoden in analoger Weise herstellen, indem man beispielsweise Ethoxymethylenmalonester der Formel (VII),

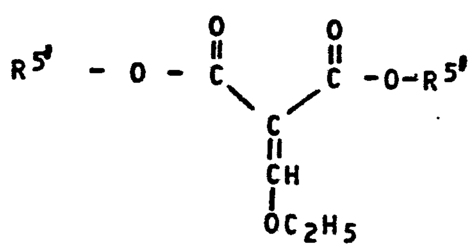

(VII)

in welcher

$R^{5'}$ die oben angegebene Bedeutung hat,

mit Amidinen bzw. Isothioharnstoffen der Formel (VIII),

$$HN = \overset{NH_2}{\overset{|}{C}} - (S)_n - R^2 \qquad \text{(VIII)}$$

in welcher

$R^2$ und n die oben angegebene Bedeutung haben,

zunächst in einer 1.Stufe gegebenenfalls in Gegenwart eines Verdünnungsmittels, wie beispielsweise Ethanol, sowie gegebenenfalls in Gegenwart eines Säurebindemittels, wie beispielsweise Kaliumhydroxid, bei Temperaturen zwischen 0°C und +120°C cyclisiert zu den Pyrimidinonen der Formel (IX),

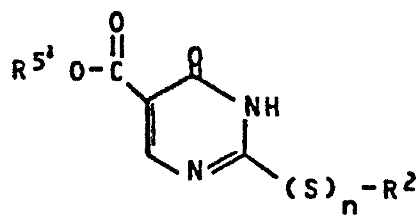

(IX)

in welcher

$R^2$, $R^{5'}$ und n die oben angegebene Bedeutung haben,

und diese in einer 2.Stufe mit einem Halogenierungsmittel, wie beispielsweise Phosphortribromid oder Phosphoroxychlorid, gegebenenfalls in Gegenwart eines Verdünnungsmittels bei Temperaturen zwischen +10°C und +150°C halogeniert.

Die Ethoxymethylenmalonsäureester der Formel (VII) und die Amidine bzw. Isothioharnstoffe der Formel (VIII) sind allgemein bekannte Verbindungen der organischen Chemie.

Die weiterhin zur Durchführung des erfindungsgemäßen Verfahrens (a) als Ausgangsstoffe benötigten aromatischen Alkohole oder Thiole sind durch die Formel (III) allgemein definiert. In dieser Formel (III) stehen $R^1$ und Y für diejenigen Reste, die schon bei der Beschreibung der erfindungsgemäßen Stoffe der Formel (I) für diese Substituenten angegeben wurden.

Die aromatischen Alkohole und Thiole der Formel (III) sind allgemein bekannte Verbindungen der organischen Chemie.

Die zur Durchführung des erfindungsgemäßen Verfahrens (b) als Ausgangsstoffe benötigten 5-Alkoxycarbonylpyrimidine sind durch die Formel (Ia) allgemein definiert. In dieser Formel (Ia) stehen $R^1$, $R^2$, $R^4$, Y und n für diejenigen Reste, die schon bei der Beschreibung der erfindungsgemäßen Stoffe der Formel (I) für diese Gruppe angegeben wurden. $R^{5'}$ steht vorzugsweise für geradkettiges oder verzweigtes Alkyl mit 1 bis 4 Kohlenstoffatomen, insbesondere für Methyl oder Ethyl. Die Ausgangsprodukte der Formel (Ia) sind erfindungsgemäße Stoffe und erhältlich nach Verfahren (a).

Die zur Durchführung der erfindungsgemäßen Verfahren (c-$\alpha$) und (c-ß) als Ausgangsstoffe benötigten Pyrimidin-5-carbonsäuren sind durch die Formel (Ib) allgemein definiert. In dieser Formel (Ib) stehen $R^1$, $R^2$, $R^4$, Y und n für diejenigen Reste, die schon bei der Beschreibung der erfindungsgemäßen Stoffe der Formel (I) für

diese Reste angegeben wurden.

Die Pyrimidin-5-carbonsäuren der Formel (Ib) sind erfindungsgemäße Stoffe und erhältlich nach Verfahren (b).

Die weiterhin zur Durchführung des erfindungsgemäßen Verfahrens (c- ) als Ausgangsstoffe benötigten Alkohole sind durch die Formel (IV) allgemein definiert. In dieser Formel (IV) steht $R^5$ für diejenigen Reste, die schon bei der Beschreibung der erfindungsgemäßen Stoffe der Formel (I) für diesen Substituenten genannt wurden.

Die Alkohole der Formel (IV) sind allgemein bekannte Verbindungen der organischen Chemie.

Die weiterhin zur Durchführung des erfindungsgemäßen Verfahrens (c-ß) als Ausgangsstoffe benötigten Amine sind durch die Formel (V) allgemein definiert. In dieser Formel (V) stehen $R^6$ und $R^7$ für diejenigen Reste, die schon bei der Beschreibung der erfindungsgemäßen Stoffe der Formel (I) für diese Substituenten genannt wurden.

Die Amine der Formel (V) sind ebenfalls allgemein bekannte Verbindungen der organischen Chemie.

Die zur Durchführung des erfindungsgemäßen Verfahrens (d) als Ausgangsstoffe benötigten Pyrimidin-Derivate sind durch die Formel (Ie) allgemein definiert. In dieser Formel (Ie) stehen $R^1$, $R^2$, $R^3$, $R^4$ und Y für diejenigen

Reste, die schon bei der Beschreibung der erfindungsgemäßen Stoffe der Formel (I) für diese Substituenten genannt wurden. Die Pyrimidin-Derivate der Formel (Ie) sind erfindungsgemäße Verbindungen und erhältlich mit Hilfe der erfindungsgemäßen Verfahren (a), (b), (c-α) oder (c-ß).

Die weiterhin zur Durchführung des erfindungsgemäßen Verfahrens (d) als Ausgangsstoffe benötigten Oxidationsmittel sind durch die Formel (VI) allgemein definiert. In dieser Formel (VI) steht R vorzugsweise für Wasserstoff, für Acetyl, für Propionyl, für Trifluoracetyl oder für gegebenenfalls substituiertes Benzoyl, wie beispielsweise 3-Chlorbenzoyl oder 4-Nitrobenzoyl. Die Oxidationsmittel der Formel (VI) sind allgemein bekannte Verbindungen der organischen Chemie.

Als Verdünnungsmittel kommen für das erfindungsgemäße Verfahren (a) inerte organische Lösungsmittel infrage. Hierzu gehören vorzugsweise aliphatische oder aromatische, gegebenenfalls halogenierte Kohlenwasserstoffe, wie beispielsweise Benzin, Benzol, Toluol, Xylol, Petrolether, Pentan, Hexan, Cyclohexan, Dichlormethan, Chloroform, Tetrachlorkohlenstoff oder Chlorbenzol; Ether, wie Diethylether, Dioxan oder Tetrahydrofuran; Ketone, wie Aceton oder Butanon; Nitrile, wie Acetonitril oder Propionitril; Amide, wie Dimethylformamid oder Hexamethylphosphorsäuretriamid oder Sulfoxide, wie Dimethylsulfoxid.

Als Säurebindemittel kommen für das erfindungsgemäße Verfahren (a) alle überlicherweise verwendbaren Säurebindemittel infrage. Vorzugsweise verwendet man Alkali- oder Erdalkalimetallhydroxide oder -carbonate, wie beispielsweise Natriumhydroxid, Natriumcarbonat oder Kaliumcarbonat, oder tertiäre Amine, wie beispielsweise Triethylamin.

Die Reaktionstemperaturen können bei der Durchführung des erfindungsgemäßen Verfahrens (a) in einem größeren Bereich variiert werden. Im allgemeinen arbeitet man zwischen 0°C und +200°C, vorzugsweise zwischen +20°C und +150°C.

Zur Durchführung des erfindungsgemäßen Verfahrens (a) setzt man pro Mol 4-Halogenpyrimidin der Formel (II) im allgemeinen 1,0 bis 1,5 Mol, vorzugsweise 1,0 bis 1,3 Mol an aromatischem Alkohol bzw. Thiol der Formel (III) und im allgemeinen 1,0 bis 1,5 Mol, vorzugsweise 1,0 bis 1,3 Mol an Säurebindemittel ein. Die Aufarbeitung und Isolierung der Reaktionsprodukte erfolgt in üblicher Art und Weise.

Für das erfindungsgemäße Verfahren (b) kommen als Verdünnungsmittel ebenfalls Wasser oder mit Wasser mischbare organische Lösungsmittel infrage. Vorzugsweise verwendet man Wasser oder Alkohol/Wasser-Gemische, wobei als Alkoholkomponente Methanol oder Ethanol besonders bevorzugt sind.

Als Basen verwendet man bei der Durchführung des erfindungsgemäßen Verfahrens (b) vorzugsweise Alkalimetallhydroxide, wie Natriumhydroxid oder Kaliumhydroxid.

Die Reaktionstemperaturen können bei dem erfindungsgemäßen Verfahren (b) in einem größeren Bereich variiert werden. Im allgemeinen arbeitet man zwischen 0°C und 100°C, vorzugsweise zwischen 20°C und 80°C.

Zur Durchführung des erfindungsgemäßen Verfahrens (b) setzt man pro Mol 5-Alkoxycarbonyl-pyrimidin der Formel (Ia) im allgemeinen 1 bis 2 Mol, vorzugsweise 1 bis 1,5 Mol an wässriger Basenlösung ein. Zur Aufarbeitung neutralisiert man mit verdünnter Salzsäure, wobei die Pyrimidin-5-carbonsäure der Formel (Ib) in der Regel auskristallisiert und durch Abfiltrieren isoliert werden kann.

Für das erfindungsgemäße Verfahren (c-$\alpha$) kommen als Verdünnungsmittel ebenfalls inerte organische Lösungsmittel infrage. Vorzugsweise verwendet man aliphatische oder aromatische, gegebenenfalls halogenierte Kohlenwasserstoffe, wie Benzin, Benzol, Toluol, Xylol, Pentan, Hexan, Heptan, Cyclohexan, Petrolether, Ligroin, Methylenchlorid, Ethylenchlorid, Chloroform, Tetrachlorkohlenstoff, Chlorbenzol oder Dichlorbenzol; Ether, wie Diethylether oder Diisopropylether, Ethylenglykoldimethylether, Tetrahydrofuran oder Dioxan; Ketone, wie Aceton oder Butanon, Methylisopropylketon oder Methylisobutylketon; Ester, wie Essigsäureethylester; Nitrile,

wie Acetonitril oder Propionitril; Amide, wie Dimethylformamid, Dimethylacetamid, N-Methylpyrrolidon oder Hexamethylphosphorsäuretriamid.

Es ist jedoch auch möglich, den als Reaktionspartner verwendeten Alkohol der Formel (IV) in entsprechendem Überschuß als Verdünnungsmittel einzusetzen.

Als Säurebindemittel können bei dem erfindungsgemäßen Verfahren (c-$\alpha$) alle üblichen Säurebindemittel Verwendung finden. Vorzugsweise verwendet man tertiäre aliphatische, aromatische oder heterocyclische Amine, wie beispielsweise Triethylamin, N,N-Dimethylanilin, N,N-Dimethylbenzylamin, Pyridin, 4-Dimethylaminopyridin, Diazabicyclooctan (DABCO), Diazabicyclononen (DBN) oder Diazabicycloundecen (DBU).

Als Aktivierungsmittel oder Katalysatoren für das erfindungsgemäße Verfahren (c-$\alpha$) lassen sich alle üblicherweise bei Veresterungen verwendeten Aktivierungsmittel oder Katalysatoren einsetzen. Hierzu gehören Protonensäuren, wie beispielsweise Schwefelsäure oder p-Toluolsulfonsäure; Lewis-Säuren, wie beispielsweise Bortrifluorid; Kondensationsmittel, wie beispielsweise N,N'-Dicyclohexylcarbodiimid (DCC) oder 2-Ethoxy-N-ethoxycarbonyl-1,2-dihydrochinolin (EEDQ); Säurehalogenidbildner, wie beispielsweise Phosphorpentachlorid, Phosphortribromid oder Thionylchlorid, oder Anhydridbildner, wie beispielsweise Chlorameisensäureethylester oder Chlorameisensäure-4-nitrophenylester.

Die Reaktionstemperaturen können bei dem erfindungsgemäßen Verfahren (c-α) in einem größeren Bereich variiert werden. Im allgemeinen arbeitet man zwischen -20°C und +150°C, vorzugsweise zwischen 0°C und +120°C.

Zur Durchführung des erfindungsgemäßen Verfahrens (c-α) setzt man pro Mol Pyrimidin-5-carbonsäure der Formel (Ib) im allgemeinen 1 bis 30 Mol, vorzugsweise 1 bis 10 Mol Alkohol der Formel (IV), 0,1 bis 2 Mol an Aktivierungsmittel oder Katalysator und 1 bis 2 Mol an Säurebindemittel ein. Die Aufarbeitung und Isolierung der Pyrimidin-5-carbonsäureester der Formel (Ic) erfolgt nach allgemein üblichen Verfahren. In einer besonderen Ausführungsform ist es auch möglich, aus der Pyrimidin-5-carbonsäure der Formel (Ib) und dem Aktivierungsmittel zunächst in einer vorgelagerten Reaktion, gegebenenfalls in Gegenwart des Säurebindemittels, einen aktivierten Komplex aus Säure und Aktivierungsmittel herzustellen und zu isolieren und diesen in einer 2.Stufe in getrennter Reaktion mit dem Alkohol der Formel (IV) gegebenenfalls in Gegenwart des Säurebindemittels umzusetzen.

Auch in diesem Fall erfolgt die Durchführung, Aufarbeitung und Isolierung der jeweiligen Endprodukte nach allgemein üblichen Verfahren (vgl. z.B. E.K. Euranto in: S. Patai "The Chemistry of Carboxylic Acids and Esters", Interscience Publishers, London 1969, S. 505 ff).

Für das erfindungsgemäße Verfahren (c-ß) kommen als Verdünnungsmittel ebenfalls inerte organische Lösungsmittel infrage. Vorzugsweise verwendet man die bei Verfahren (c-α) genannten Lösungsmittel.

Als Säurebindemittel kommen für das erfindungsgemäße Verfahren (c-ß) alle üblicherweise verwendbaren anorganischen und organischen Basen infrage. Vorzugsweise verwendet man Alkalimetallhydroxide oder -carbonate, wie beispielsweise Natriumhydroxid, Kaliumhydroxid, Natriumcarbonat oder Kaliumcarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DNU).

Es ist jedoch auch möglich, einen entsprechenden Überschuß des als Reaktionspartner eingesetzten Amins der Formel (V) als Säurebindemittel zu verwenden.

Als Aktivierungsmittel für das erfindungsgemäße Verfahren (c-ß) lassen sich alle üblicherweise bei Amidierungsreaktionen verwendeten Aktivierungsmittel einsetzen. Beispielhaft seien Säurehalogenidbildner, wie Phosphortribromid, Phosphorpentachlorid, Phosphoroxychlorid oder Thionylchlorid, Anhydridbildner, wie Chlorameisensäureethylester, sowie Kondensationsmittel, wie N,N'-Dicyclohexylcarbodiimid (DCC), 2-Ethoxy-N-ethoxycarbonyl-1,2-dihydrochinolin (EEDQ) oder Triphenylphosphin/Tetrachlorkohlenstoff genannt.

Die Reaktionstemperaturen können bei dem erfindungsgemäßen Verfahren (c-ß) in einem größeren Bereich variiert werden. Im allgemeinen arbeitet man zwischen -20°C und +220°C, vorzugsweise zwischen 0°C und +150°C.

Zur Durchführung des erfindungsgemäßen Verfahrens (c-ß) setzt man pro Mol Pyrimidin-5-carbonsäure der Formel (Ib) im allgemeinen 1 bis 5 Mol, vorzugsweise 1 bis 3 Mol an Amin der Formel (V), sowie 1 bis 2 Mol an Säurebindemittel und 1 bis 2 Mol an Aktivierungsmittel ein. Die Aufarbeitung und Isolierung der Pyrimidin-5-carbonsäureamide der Formel (Id) erfolgt nach allgemein üblichen Verfahren. Wie bei der Durchführung des erfindungsgemäßen Verfahrens (c-$\alpha$) ist es auch hierbei in einer besonderen Ausführungsform möglich, aus der Pyrimidin-5-carbonsäure der Formel (Ib) und dem Aktivierungsmittel zunächst in einer vorgelagerten Reaktion, gegebenenfalls in Gegenwart des Säurebindemittels, einen aktivierten Komplex aus Säure und Aktivierungsmittel herzustellen und zu isolieren und diesen in einer 2.Stufe in getrennter Reaktion mit dem Amin der Formel (V), gegebenenfalls in Gegenwart des Säurebindemittels, umzusetzen. Auch in diesem Fall erfolgt die Durchführung, Aufarbeitung und Isolierung der jeweiligen Endprodukte nach allgemein üblichen Verfahren.

Als Verdünnungsmittel zur Durchführung des erfindungsgemäßen Verfahrens (d) kommen ebenfalls inerte organische

Lösungsmittel infrage.

Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenwasserstoff oder Chlorbenzol; Ether, wie Diethylether, Dioxan oder Tetrahydrofuran; Carbonsäuren, wie Essigsäure oder Propionsäure, oder dipolar aprotische Lösungsmittel, wie Acetonitril, Aceton, Essigsäureethylester oder Dimethylformamid.

Das erfindungsgemäße Verfahren (d) wird gegebenenfalls in Gegenwart eines Säurebindemittels durchgeführt. Als solche kommen alle üblicherweise verwendbaren organischen und anorganischen Säurebindemittel infrage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Die Reaktionstemperaturen können bei dem erfindungsgemäßen Verfahren (d) in gewissen Bereichen variiert werden. Im allgemeinen arbeitet man zwischen -20°C und 70°C, vorzugsweise zwischen 0°C und 50°C.

Zur Durchführung des erfindungsgemäßen Verfahrens (d) setzt man pro Mol des Pyrimidin-Derivates der Formel (Ie) im allgemeinen 0,8 bis 1,5 Mol, vorzugsweise äquimolare Mengen an Oxidationsmittel der Formel (VI) ein. Die Aufarbeitung und Isolierung der 2-Sulfonylpyrimidine der Formel (If) erfolgt in allgemein üblicher Art und Weise.

Zur Herstellung von pflanzenverträglichen Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblicher Salzbildungsmethoden, wie z.B. durch Lösen einer Verbindung der Formel (I) in einem geeigneten inerten Lösungsmittel und hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Cornybacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis), von Obstkrankheiten, wie beispielsweise gegen den Erreger des Apfelmehltaus (Podosphaera leucotricha) oder von Reiskrankheiten, wie beispielsweise gegen den Erreger der Reisfleckenkrankheit (Pyricularia oryzae) oder gegen den Erreger des Bakterienbrandes (Xanthomonas oryzae) eingesetzt werden.

Dabei zeigen die erfindungsgemäßen Wirkstoffe neben der protektiven Wirksamkeit auch eine systemische Wirkung.

Die Wirkstoffe können in die üblichen Formulierungen überführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen infrage: Aromaten, wie Xylol, Toluol oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe wie Cyclohexan oder Paraffine, z.B. Erdölfraktionen, Alkohole, wie Butanol oder Glykol sowie deren Ether und Ester, Ketone,

wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoff sowie Butan, Propan, Stickstoff und Kohlendioxid; als feste Trägerstoffe kommen infrage; z.B. natürliche Gesteinsmehle, wie Kaoline, Tonerden, Talkum, Kreide, Quarz, Attapulgit, Montmorillonit oder Diatomenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden,

wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten, und Granulate

angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Herstellungsbeispiele

Beispiel 1

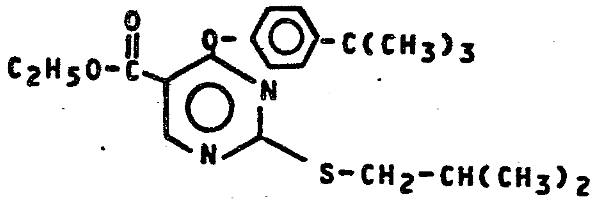

(Verfahren a)

Zu einer Lösung von 78,1 g (0,52 Mol) 4-t-Butylphenol in 200 ml Aceton gibt man unter Rühren zunächst eine Lösung von 72,5 g (0,52 Mol) Triethylamin in Aceton und danach 150,4 g (0,55 Mol) 4-Chlor-5-ethoxycarbonyl-2-isobutylthiopyrimidin in 100 ml Aceton. Nach beendeter Zugabe rührt man 90 Stunden bei Raumtemperatur, gießt dann die Mischung in 1500 ml Wasser, extrahiert mehrfach mit Dichlormethan, wäscht die vereinigten organischen Phasen dreimal mit Wasser, trocknet über Natriumsulfat und engt im Vakuum ein. Man erhält 204,9 g (94,7 % der Theorie) an 4-(4-t-Butylphenoxy)-5-ethoxycarbonyl-2-isobutylthiopyrimidin als Öl.
$^1$H-NMR($CDCl_3$) : $\delta$ = 8,90 ppm.

Herstellung der Ausgangsverbindung

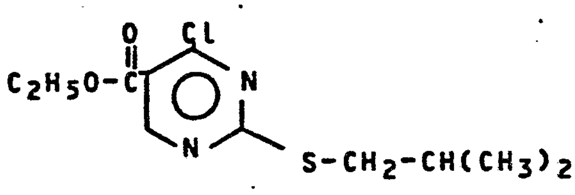

76,35 g (0,3 Mol) 5-Ethoxycarbonyl-2-isobutylthiopyrimidin-4-on werden bei Raumtemperatur tropfenweise mit 10 ml Phosphoroxychlorid versetzt. Während die Reaktionsmischung langsam auf 120°C aufgeheizt wird, tropft man weitere 25 ml Phosphoroxychlorid zu. Nach beendeter Chlorwasserstoffentwicklung gibt man 200 ml Dichlormethan zu der erhaltenen Reaktionsmischung und gießt sie portionsweise in 500 ml Wasser. Die organische Phase wird abgetrennt, mehrfach mit Wasser neutral gewaschen, über Natriumsulfat getrocknet und im Vakuum eingeengt. Man erhält 75,2 g (91,2 % der Theorie) an 4-Chlor-5-ethoxycarbonyl-2-isobutylthiopyrimidin als Öl.
$^1H$-NMR($CDCl_3$) = $\delta$ 8,92 ppm.

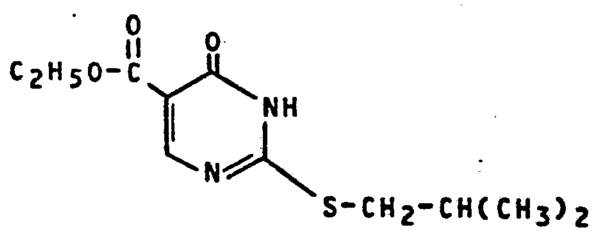

190 g (2,5 Mol) Thioharnstoff und 272 ml (2,5 Mol) Isobutylbromid in 250 ml Ethanol werden 9 Stunden lang am Rückflußkühler gekocht und nach dem Abkühlen zunächst bei Raumtemperatur tropfenweise mit einer Lösung von 464,3 ml (2,3 Mol) Ethoxymethylenmalonsäurediethylester versetzt und danach ebenfalls tropfenweise unter Eiskühlung mit einer Lösung von 280 g (5 Mol) Kaliumhydroxid in 800 ml Wasser versetzt, 2 Stunden bei Raumtemperatur gerührt, tropfenweise mit 170 ml Eisessig neutralisiert und zweimal mit Dichlormethan extrahiert. Die vereinigten organischen Phasen werden zweimal mit Wasser gewaschen, über Natriumsulfat getrocknet und im Vakuum eingeengt. Der kristalline Rückstand läßt sich aus Cyclohexan umkristallisieren. Man erhält 404,3 g (68,7 % der Theorie) an 5-Ethoxycarbonyl-2-isobutylthio-pyrimidin-4-on vom Schmelzpunkt 105-106°C.

Beispiel 2

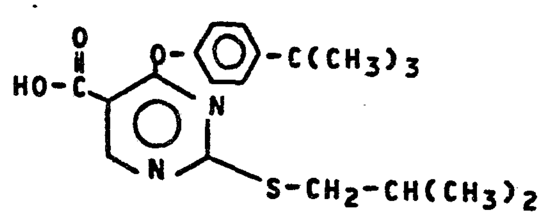

(Verfahren b)

20 g (0,05 Mol) 4-(4-t-Butylphenoxy)-5-ethoxycarbonyl-2-isobutylthiopyrimidin in 50 ml 80 %igem wäßrigem Ethanol werden bei Raumtemperatur unter Rühren tropfenweise mit einer Lösung von 2,8 g (0,05 Mol) Kaliumhydroxid in 100 ml 80 %igem wäßrigem Ethanol versetzt und nach beendeter Zugabe weitere 60 Minuten bei Raumtemperatur gerührt. Zur Aufarbeitung säuert man mit verdünnter wäßriger Salzsäure an, saugt den kristallinen

Niederschlag ab, wäscht mit Petrolether nach und trocknet den so erhaltenen Feststoff. Man erhält 17,6 g (97,8 % der Theorie) an 4-(4-t-Butylphenoxy)-2-isobutylthio-pyrimidin-5-carbonsäure vom Schmelzpunkt 160°C-164°C.

Beispiel 3

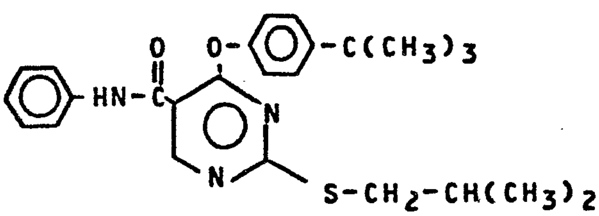

(Verfahren c-ß)

10,8 g (0,03 Mol) an 4-(4-t-Butylphenoxy)-2-isobutylthio-pyrimidin-5-carbonsäure werden tropfenweise mit 20 ml Thionylchlorid versetzt, nach 12 Stunden bei Raumtemperatur im Vakuum eingeengt, das verbleibende Öl in 100 ml Dioxan gelöst, tropfenweise mit 5,3 g (0,06 Mol) Anilin versetzt, nach beendeter Zugabe 2 Stunden bei Raumtemperatur gerührt, zur Aufarbeitung auf Wasser gegossen, dreimal mit Methylenchlorid extrahiert, die vereinigten organischen Phasen zweimal mit Wasser gewaschen, über Natriumsulfat getrocknet und im Vakuum eingeengt. Das verbleibende Öl kristallisiert nach mehreren Stunden und läßt sich dann aus Ethanol umkristallisieren. Man

erhält 6,0 g (46 % der Theorie) an 4-(4-t-Butylphenoxy)-2-isobutylthiopyrimidin-5-carbonsäureanilid vom Schmelzpunkt 118-121°C.

Beispiel 4

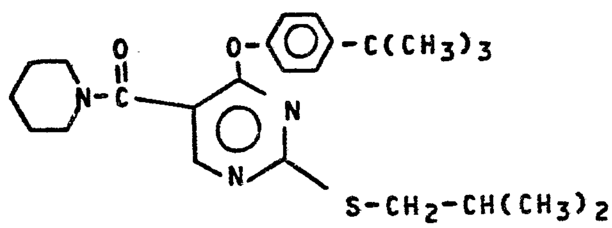

(Verfahren c-ß)

7,4 g (0,02 Mol) 4-(4-t-Butylphenoxy)-2-isobutylthiopyrimidin-5-carbonsäure werden tropfenweise mit 20 ml Thionylchlorid versetzt, nach 12 Stunden bei Raumtemperatur im Vakuum von flüchtigen Anteilen befreit, der Rückstand in 70 ml Dioxan aufgenommen, tropfenweise mit 6,0 ml (0,06 Mol) Piperidin in 30 ml Dioxan versetzt, 2 Stunden bei Raumtemperatur gerührt, zur Aufarbeitung in Wasser gegossen und der kristalline Niederschlag abfiltriert und getrocknet. Man erhält 8,1 g (94,9 % der Theorie) an 4-(4-t-Butylphenoxy)-2-isobutylthiopyrimidin-5-carbonsäurepiperidid vom Schmelzpunkt 115°C.

In entsprechender Weise und gemäß den allgemeinen Herstellungsangaben erhält man die folgenden Verbindungen der allgemeinen Formel (I)

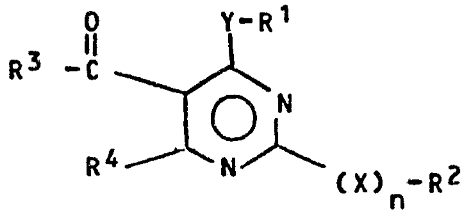

(I)

| Bsp.Nr. | $R^1$ | $R^2$ | $R^3$ | $R^4$ | X | Y | n | physikal. Daten |
|---|---|---|---|---|---|---|---|---|
| 5 | 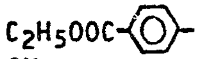 | $CH_3$ | $C_2H_5O$ | H | S | O | 1 | Fp:91-93°C |
| 6 | 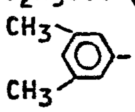 | $C_2H_5$ | $C_2H_5O$ | H | S | O | 1 | Fp:44-46°C |
| 7 | 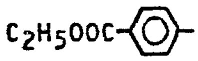 | $C_2H_5$ | $C_2H_5O$ | H | S | O | 1 | Fp:76-78°C |
| 8 | 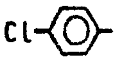 | $C_2H_5$ | $C_2H_5O$ | H | S | O | 1 | Fp:58-59°C |
| 9 | 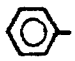 | 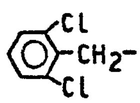 | $C_2H_5O$ | H | S | O | 1 | Fp:91-93°C |
| 10 | 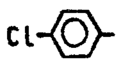 | 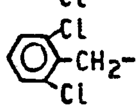 | $C_2H_5O$ | H | S | O | 1 | Fp:102-105°C |
| 11 | 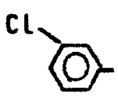 | 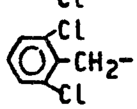 | $C_2H_5O$ | H | S | O | 1 | Fp:81-83°C |
| 12 | 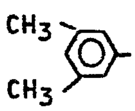 | $CH_3$ | $C_2H_5O$ | H | S | O | 1 | Fp:75-77°C |
| 13 | 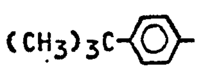 | $CH_3$ | $C_2H_5O$ | H | S | O | 1 | Fp:87-89°C |
| 14 | 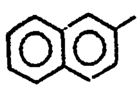 | $CH_3$ | $C_2H_5O$ | H | S | O | 1 | Fp:94-97°C |
| 15 | 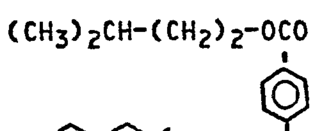 | $CH_3$ | $C_2H_5O$ | H | S | O | 1 | Fp:99-101°C |
| 16 | 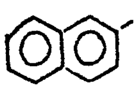 | $C_2H_5$ | $C_2H_5O$ | H | S | O | 1 | Oel |
| 17 | 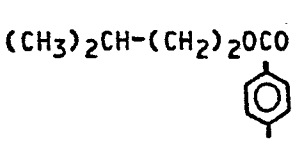 | $C_2H_5$ | $C_2H_5O$ | H | S | O | 1 | Fp:46-48°C |

| Bsp.Nr. | $R^1$ | $R^2$ | $R^3$ | $R^4$ | X | Y | n | physikal. Daten |
|---|---|---|---|---|---|---|---|---|
| 18 | 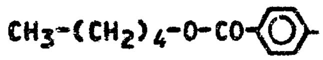 | $C_2H_5$ | $C_2H_5O$ | H | S | 0 | 1 | Fp:78-81°C |
| 19 | 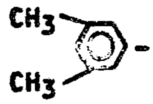 | $C_2H_5$ | $C_2H_5O$ | H | S | 0 | 1 | Fp:69°C |
| 20 | 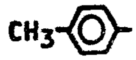 | $C_2H_5$ | $C_2H_5O$ | H | S | 0 | 1 | Oel |
| 21 | 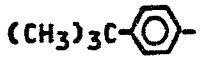 | $C_2H_5$ | $C_2H_5O$ | H | S | 0 | 1 | Oel |
| 22 | 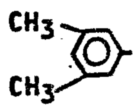 | $(CH_3)_2CH-CH_2-$ | $C_2H_5O$ | H | S | 0 | 1 | Fp:64-65°C |
| 23 | 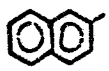 | $(CH_3)_2CH-CH_2-$ | $C_2H_5O$ | H | S | 0 | 1 | Fp:63-64°C |
| 24 | 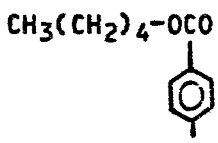 | $(CH_3)_2CH-CH_2-$ | $C_2H_5O$ | H | S | 0 | 1 | Fp:50°C |
| 25 | 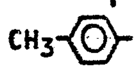 | $(CH_3)_2CH-CH_2-$ | $C_2H_5O$ | H | S | 0 | 1 | Oel |
| 26 | 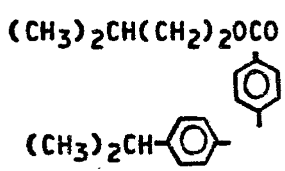 | $(CH_3)_2CH-CH_2-$ | $C_2H_5O$ | H | S | 0 | 1 | Oel |
| 27 | | $(CH_3)_2CH-CH_2-$ | $C_2H_5O$ | H | S | 0 | 1 | Oel |
| 28 | 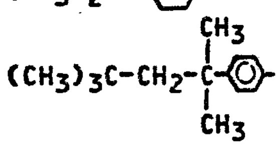 | $(CH_3)_2CH-CH_2-$ | $C_2H_5O$ | H | S | 0 | 1 | Kp 220°C/0,13 mm Hg |
| 29 | 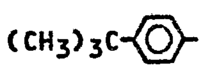 | $(CH_3)_2CH-CH_2$ | $C_2H_5O$ | H | S | 0 | 1 | Fp:120°C [xHCl] |
| 30 | 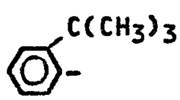 | $(CH_3)_2CH-CH_2-$ | $C_2H_5O$ | H | S | 0 | 1 | Fp:89-93°C |
| 31 | 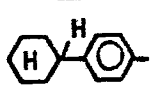 | $(CH_3)_2CH-CH_2-$ | $C_2H_5O$ | H | S | 0 | 1 | Fp:63°C |
| 32 | 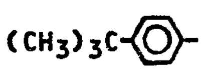 | $(CH_3)_2CH-CH_2-$ | $CH_3(CH_2)_3NH-$ | H | S | 0 | 1 | Fp:58-60°C |
| 33 | 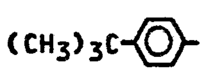 | $CH_3-(CH_2)_3-$ | $C_2H_5O$ | H | S | 0 | 1 | Kp 200°C/0,1 mm Hg |

| Bsp.Nr. | $R^1$ | $R^2$ | $R^3$ | $R^4$ | X | Y | n | physikal. Daten |
|---|---|---|---|---|---|---|---|---|
| 34 | $(CH_3)_3C$-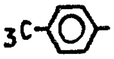- | $(CH_3)_2CH$- | $C_2H_5O$ | H | S | O | 1 | Kp 205°C/0,1 mm H |
| 35 | $(CH_3)_3C$-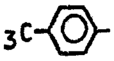- | $(CH_3)_2CH-CH_2$- | $CH_3O$-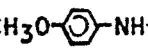-NH- | H | S | O | 1 | Fp 140°C |
| 36 | $(CH_3)_3C$-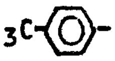- | $(CH_3)_2CH-CH_2$- | Cl-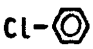-NH- | H | S | O | 1 | Fp 127°C |
| 37 | $(CH_3)_3C$-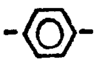- | $CH_3-(CH_2)_5$ | $C_2H_5O$ | H | S | O | 1 | Kp: 240°C/0,1 mm Hg |
| 38 | $(CH_3)_3C$-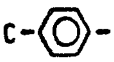- | $CH_2=CH-CH_2$- | $C_2H_5O$ | H | S | O | 1 | Kp: 230°C/0,15 mm Hg |
| 39 | $(CH_3)_3C$-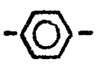- | $(CH_3)_2CH-CH_2$- | $C_2H_5O$ | $CH_3$ | S | O | 1 | Fp:71-73°C |
| 40 | $(CH_3)_3C$-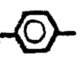- | 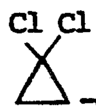-$CH_2$- | $C_2H_5O$ | H | S | O | 1 | Kp: 240°C/0,1 mm Hg |
| 41 | $(CH_3)_3C$-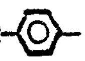- | $CH_3-(CH_2)_2$- | $C_2H_5O$ | H | S | O | 1 | Kp: 230°C/0,1 mm Hg |
| 42 | $(CH_3)_3C$-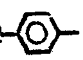- | $(CH_3)_2CH-CH_2$- | $NH_2$ | H | S | O | 1 | Fp 170°C |
| 43 | $(CH_3)_3C$-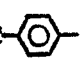- | $(CH_3)_2CH-CH_2$- | $(CH_3)_2CH-CH_2-O$- | H | S | O | 1 | Kp: 250°C/0,1 mm Hg |
| 44 | 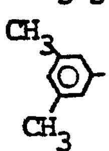 | $CH_3-(CH_2)_2$- | $C_2H_5O$ | H | S | O | 1 | Kp: 195°C/0,1 mm Hg |
| 45 | 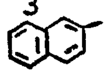 | $CH_3-(CH_2)_2$- | $C_2H_5O$ | H | S | O | 1 | Fp. 72°C |
| 46 | $(CH_3)_3C$-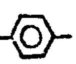- | $C_6H_5-CH_2$- | $C_2H_5O$ | H | S | O | 1 | Öl |

| Bsp.Nr. | $R^1$ | $R^2$ | $R^3$ | $R^4$ | X | Y | n | physikalische Daten |
|---|---|---|---|---|---|---|---|---|
| 47 | 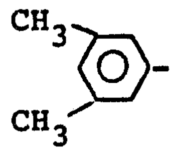 | $CH_3-(CH_2)_5-$ | $C_2H_5O$ | H | S | O | 1 | Fp. 52°C |
| 48 | 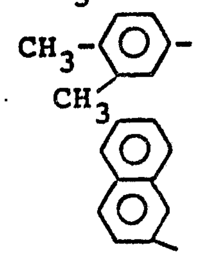 | $CH_3-(CH_2)_5-$ | $C_2H_5O$ | H | S | O | 1 | $Kp_{0,1}$: 220°C |
| 49 | | $CH_3(CH_2)_5-$ | $C_2H_5O$ | H | S | O | 1 | Fp 70°C |
| 50 | 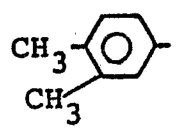 | $CH_3-(CH_2)_2-$ | $C_2H_5O$ | H | S | O | 1 | $Kp_{0,1}$: 195°C |
| 51 | 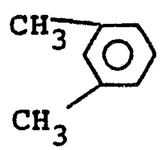 | $CH_3-(CH_2)_3-$ | $C_2H_5O$ | H | S | O | 1 | Fp 62°C |
| 52 | 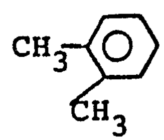 | $CH_3-(CH_2)_3-$ | $C_2H_5O$ | H | S | O | 1 | $Kp_{0,1}$: 190°C |

| Bsp. Nr. | $R^1$ | $R^2$ | $R^3$ | $R^4$ | X | Y | n | physikalische Daten |
|---|---|---|---|---|---|---|---|---|
| 53 | 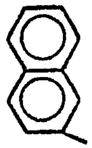 | $CH_3-(CH_2)_3-$ | $C_2H_5O$ | H | S | O | 1 | Fp 66°C |
| 54 | 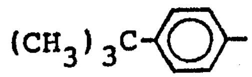 | $(CH_3)CH-CH_2-$ | $C_2H_5O$ | H | S | S | 1 | Fp 70°-72°C |
| 55 | 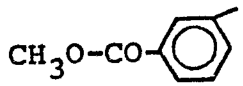 | $(CH_3)_2CH-CH_2-$ | $C_2H_5O$ | H | S | O | 1 | Fp 55°C |
| 56 | 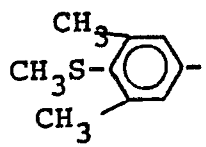 | $(CH_3)_2CH-CH_2-$ | $C_2H_5O$ | H | S | O | 1 | Fp 52°C |
| 57 | 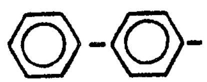 | $(CH_3)_2CH-CH_2-$ | $C_2H_5O$ | H | S | O | 1 | Fp 74°C |
| 58 | 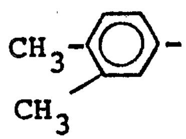 | $(CH_3)_2CH-CH_2-$ | $C_2H_5O$ | H | S | O | 1 | $Kp_{0,1}$: 190°C |
| 59 | 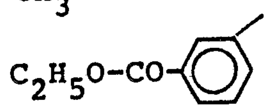 | $(CH_3)_2CH-CH_2-$ | $C_2H_5O$ | H | S | O | 1 | Fp 55°C |
| 60 | 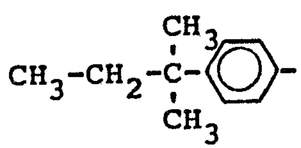 | $(CH_3)_2CH-CH_2-$ | $C_2H_5O$ | H | S | O | 1 | $Kp_{0,1}$: 225°C |

| Bsp. Nr. | $R^1$ | $R^2$ | $R^3$ | $R^4$ | X | Y | n | physikalische Daten |
|---|---|---|---|---|---|---|---|---|
| 61 | 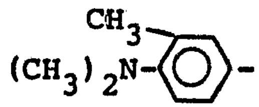 | $(CH_3)_2CH-CH_2-$ | $C_2H_5O$ | H | S | O | 1 | $Kp_{0,1}$: 190°C |
| 62 | 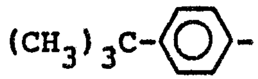 | $(CH_3)_2CH-CH_2-$ | $(CH_3)_2CH-O-$ | H | S | O | 1 | $Kp_{0,1}$: 210°C |
| 63 | 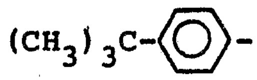 | $(CH_3)_2CH-CH_2-$ | $CH_3O$ | H | S | O | 1 | $Kp_{0,1}$: 190°C |
| 64 | 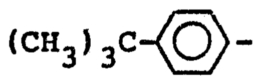 | $(CH_3)_2CH-CH_2-$ | $(CH_3)_2N-$ | H | S | O | 1 | Fp 90°C |
| 65 | 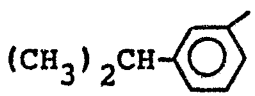 | $(CH_3)_2CH-CH_2-$ | $C_2H_5O$ | H | S | O | 1 | $Kp_{0,1}$: 170°C |
| 66 | 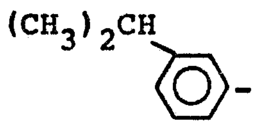 | $CH_3-(CH_2)_3-$ | $C_2H_5O$ | H | S | O | 1 | $Kp_{0,1}$: 215°C |
| 67 | 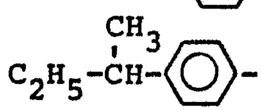 | $CH_3-(CH_2)_3-$ | $C_2H_5O$ | H | S | O | 1 | $Kp_{0,1}$: 205°C |
| 68 | 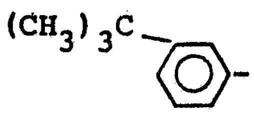 | $(CH_3)_2CH-CH_2-$ | $C_2H_5O$ | H | S | O | 1 | $Kp_{0,1}$: 215°C |
| 69 | 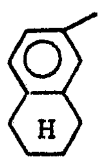 | $(CH_3)_2CH-CH_2-$ | $C_2H_5O$ | H | S | O | 1 | $Kp_{0,1}$: 230°C |

| Bsp. Nr. | $R^1$ | $R^2$ | $R^3$ | $R^4$ | X | Y | n | physikalische Daten |
|---|---|---|---|---|---|---|---|---|
| 70 | 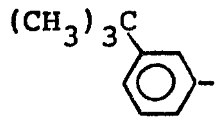 | $CH_3-(CH_2)_3-$ | $C_2H_5O$ | H | S | O | 1 | $Kp_{0,1}$: 205°C |
| 71 | 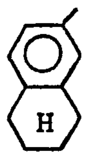 | $CH_3-(CH_2)_3-$ | $C_2H_5O$ | H | S | O | 1 | $Kp_{0,1}$: 210°C |
| 72 | 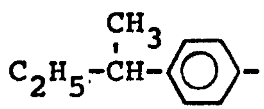 | $(CH_3)_2CH-CH_2-$ | $C_2H_5O$ | H | S | O | 1 | $Kp_{0,1}$: 200°C |
| 73 | 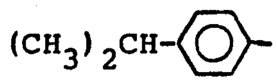 | $CH_3-(CH_2)_3-$ | $C_2H_5O$ | H | S | O | 1 | $Kp_{0,1}$: 200°C |
| 74 | 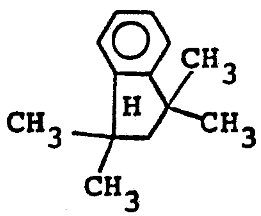 | $(CH_3)_2CH-CH_2-$ | $C_2H_5O$ | H | S | O | 1 | Fp 87-89°C |
| 75 | 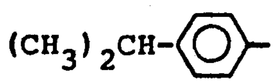 | $CH_3-(CH_2)_5-$ | $C_2H_5O$ | H | S | O | 1 | $Kp_{0,1}$: 225°C |
| 76 | 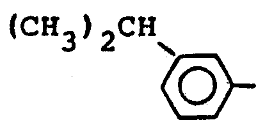 | $CH_3-(CH_2)_5-$ | $C_2H_5O$ | H | S | O | 1 | $Kp_{0,1}$: 230°C |

| Bsp. Nr. | $R^1$ | $R^2$ | $R^3$ | $R^4$ | X | Y | n | physikalische Daten |
|---|---|---|---|---|---|---|---|---|
| 77 | 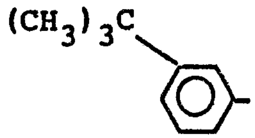 | $CH_3-(CH_2)_5-$ | $C_2H_5O$ | H | S | O | 1 | $Kp_{0,1}$: 220°C |
| 78 | 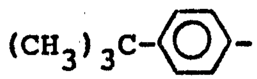 | $CH_3-(CH_2)_2-$ | OH | H | S | O | 1 | Fp: 190°C |
| 79 | 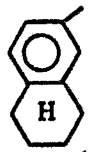 | $CH_3-(CH_2)_5-$ | $C_2H_5O$ | H | S | O | 1 | $Kp_{0,1}$: 220°C |
| 80 | 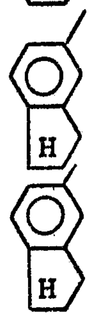 | $(CH_3)_2CH-CH_2-$ | $C_2H_5O$ | H | S | O | 1 | $Kp_{0,1}$: 220°C |
| 81 | | $CH_3-(CH_2)_2-$ | $C_2H_5O$ | H | S | O | 1 | $Kp_{0,1}$: 220°C |
| 82 | 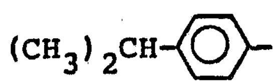 | $CH_3-(CH_2)_2-$ | $C_2H_5O$ | H | S | O | 1 | $Kp_{0,1}$: 200°C |
| 83 | 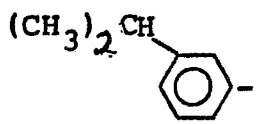 | $CH_3-(CH_2)_2-$ | $C_2H_5O$ | H | S | O | 1 | $Kp_{0,1}$: 200°C |

| Bsp. Nr. | $R^1$ | $R^2$ | $R^3$ | $R^4$ | X | Y | n | physikalische Daten |
|---|---|---|---|---|---|---|---|---|
| 84 | 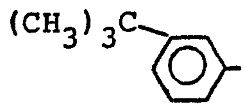 | $CH_3-(CH_2)_2-$ | $C_2H_5O$ | H | S | O | 1 | $Kp_{0,1}$: 220°C |
| 85 | 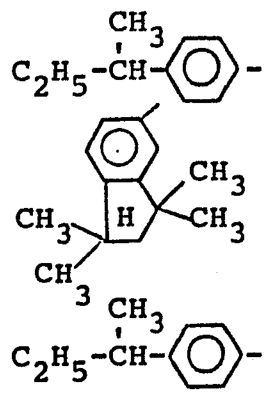 | $CH_3-(CH_2)_2-$ | $C_2H_5O$ | H | S | O | 1 | $Kp_{0,1}$: 200°C |
| 86 |  | $CH_3-(CH_2)_3-$ | $C_2H_5O$ | H | S | O | 1 | $Kp_{0,1}$: 210°C |
| 87 |  | $CH_3-(CH_2)_5-$ | $C_2H_5O$ | H | S | O | 1 | $Kp_{0,1}$: 210°C |
| 88 | 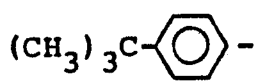 | $CH_3-(CH_2)_2-$ | $CH_3O$ | H | S | O | 1 | Fp 80°C |

Anwendungsbeispiele

In den folgenden Anwendungsbeispielen werden die nachstehend aufgeführten Verbindungen als Vergleichssubstanzen eingesetzt:

(A) 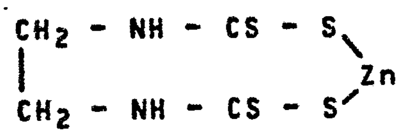

Zink-ethylen-1,2-bis-(dithiocarbamat)

(B) 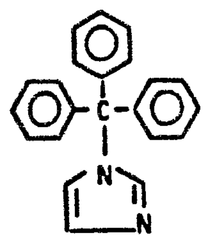

1-Triphenylmethylimidazol

Beispiel A

Podosphaera-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23°C und einer relativen Luftfeuchtigkeit von ca. 70 % aufgestellt.

9 Tage nach der Inokulation erfolgt die Auswertung.

Eine deutliche Überlegenheit in der Wirksamkeit gegenüber dem Stand der Technik zeigen in diesem Test z.B. die Verbindungen gemäß Herstellungsbeispielen: 1 und 21.

Beispiel B

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß der Herstellungsbeispiele: 14 und 16.

Beispiel C

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigt in diesem Test z.B. die Verbindung gemäß dem Herstellungsbeispiel: 19.

Beispiel D

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele: 1 und 21.

Patentansprüche

1. Substituierte Pyrimidine der allgemeinen Formel (I)

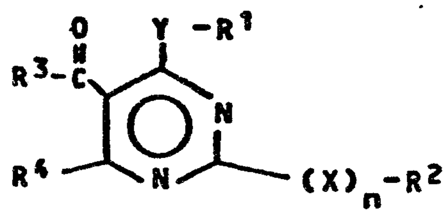

(I)

in welcher

$R^1$ für gegebenenfalls substituiertes Aryl steht,

$R^2$ für Alkyl, für Alkenyl, für gegebenenfalls substituiertes Cycloalkylalkyl oder für gegebenenfalls substituiertes Aralkyl steht,

$R^3$ für Hydroxy, für einen Rest $-O-R^5$ oder für

einen Rest 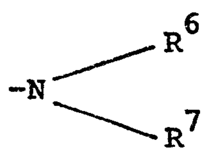 steht,

wobei $R^5$ für Alkyl, für gegebenenfalls substituiertes Cycloalkylalkyl oder für gegebenenfalls substituiertes Aralkyl steht und

$R^6$ und $R^7$ unabhängig voneinander für Wasserstoff, für Alkyl oder für gegebenenfalls substituiertes Aryl stehen oder

$R^6$ und $R^7$ gemeinsam mit dem Stickstoffatom, an welches sie gebunden sind, für einen gegebenenfalls substituierten gesättigten Heterocyclus stehen, der weitere Heteroatome enthalten kann,

$R^4$ für Wasserstoff oder Alkyl steht,

X für Schwefel oder die Sulfonylgruppe steht,

Y für Sauerstoff oder Schwefel steht,

n für 0 oder 1 steht,

wobei $R^1$ nur dann für unsubstituiertes Phenyl steht, wenn nicht gleichzeitig $R^2$ für Methyl, n für 1 und X für Schwefel stehen, und wobei $R^1$ nur dann für 2-Nitrophenyl steht, wenn nicht gleichzeitig Y für Schwefel, n für 0 und $R^2$ für Methyl stehen, sowie deren pflanzenverträgliche Säureadditionssalze.

2. Substituierte Pyrimidine der Formel (I) gemäß Anspruch 1, wobei

$R^1$ für gegebenenfalls einfach oder mehrfach, gleich oder verschieden substituiertes Aryl mit 6 bis 10 Kohlenstoffatomen steht, wobei als Substituenten infrage kommen: Halogen, jeweils geradkettiges oder verzweigtes, gegebenenfalls einfach oder mehrfach, gleich oder verschieden durch Halogen substituiertes Alkyl, Alkoxy oder Alkylthio mit jeweils 1 bis 12 Kohlenstoffatomen, gegebenenfalls einfach oder mehrfach, gleich oder verschieden durch geradkettiges oder verzweigtes Alkyl mit 1 bis 4 Kohlenstoffatomen substituiertes zweifach verknüpftes Alkylen mit 3 bis 5 Kohlenstoffatomen, gegebenenfalls einfach oder mehrfach, gleich oder verschieden durch Halogen oder geradkettiges oder verzweigtes Alkyl mit 1 bis 4 Kohlenstoffatomen substituiertes Cycloalkyl mit 3 bis 7 Kohlenstoffatomen, Amino, N-Alkylamino oder N,N-Dialkylamino mit jeweils 1 bis 4 Kohlenstoffatomen in den einzelnen geradkettigen oder verzweigten Alkylteilen, Alkoxycarbonyl mit 1 bis 10 Kohlenstoffatomen im geradkettigen oder verzweigten Alkylteil oder Phenyl,

$R^2$ für jeweils geradkettiges oder verzweigtes Alkyl oder Alkenyl mit jeweils bis zu 10 Kohlenstoffatomen, für gegebenenfalls einfach oder mehrfach, gleich oder verschieden durch Halogen, geradkettiges oder verzweigtes Alkyl mit 1 bis 4 Kohlenstoffatomen im Cycloalkylteil substituiertes Cycloalkylalkyl mit 1 bis 4 Kohlenstoffatomen im geradkettigen oder verzweigten Alkylteil und 3 bis 7 Kohlenstoffatomen im Cycloalkylteil,

oder für gegebenenfalls einfach oder mehrfach, gleich oder verschieden durch Halogen oder jeweils geradkettiges oder verzweigtes Alkyl oder Alkoxy mit jeweils 1 bis 4 Kohlenstoffatomen im Arylteil substituiertes Aralkyl mit 1 bis 4 Kohlenstoffatomen im geradkettigen oder verzweigten Alkylteil und 6 bis 10 Kohlenstoffatomen im Arylteil steht,

$R^3$ für Hydroxy, für einen Rest $-O-R^5$ oder für einen Rest 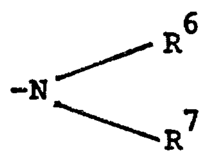 steht, wobei

$R^5$ für geradkettiges oder verzweigtes Alkyl mit 1 bis 10 Kohlenstoffatomen oder für gegebenenfalls einfach oder mehrfach, gleich oder verschieden durch Halogen, geradkettiges oder verzweigtes Alkyl mit 1 bis 4 Kohlenstoffatomen im Cycloalkylteil substituiertes Cycloalkylalkyl mit 1 bis 4 Kohlenstoffatomen im geradkettigen oder verzweigten Alkylteil und 3 bis 7 Kohlenstoffatomen im Cycloalkylteil, oder für gegebenenfalls einfach oder mehrfach, gleich oder verschieden durch Halogen oder jeweils geradkettiges oder verzweigtes Alkyl oder Alkoxy mit jeweils 1 bis 4 Kohlenstoffatomen im Arylteil substituiertes Aralkyl mit 1 bis 4 Kohlenstoffatomen im geradkettigen oder verzweigten Alkylteil und 6 bis 10 Kohlenstoffatomen im Arylteil steht, und

$R^6$ und $R^7$ unabhängig voneinander jeweils für Wasserstoff, für geradkettiges oder verzweigtes Alkyl mit 1 bis 10 Kohlenstoffatomen oder für gegebenenfalls einfach oder mehrfach, gleich oder verschieden durch Halogen oder jeweils geradkettiges oder verzweigtes Alkyl oder Alkoxy mit jeweils 1 bis 4 Kohlenstoffatomen substituiertes Aryl mit 6 bis 10 Kohlenstoffatomen stehen oder

$R^6$ und $R^7$ gemeinsam mit dem Stickstoffatom, an welches sie gebunden sind, für einen gegebenenfalls einfach oder mehrfach, gleich oder verschieden durch Alkyl mit 1 bis 4 Kohlenstoffatomen substituierten gesättigten 5- bis 7-gliedrigen Heterocyclus stehen, der ein Stickstoff- oder Sauerstoffatom als weiteres Heteroatom enthalten kann,

$R^4$ für Wasserstoff oder für geradkettiges oder verzweigtes Alkyl mit 1 bis 8 Kohlenstoffatomen steht,

X für Schwefel oder die Sulfonylgruppe steht,

Y für Sauerstoff oder Schwefel steht und

n für 0 oder 1 steht,

wobei $R^1$ nur dann für unsubstituiertes Phenyl steht, wenn nicht gleichzeitig $R^2$ für Methyl, n für 1 und X für Schwefel stehen und wobei $R^1$ nur dann für 2-Nitrophenyl steht, wenn nicht gleichzeitig Y für Schwefel, n für 0 und $R^2$ für Methyl stehen.

3. Substituierte Pyrimidine gemäß Anspruch 1, wobei in der Formel (I)

$R^1$ für gegebenenfalls ein- bis fünffach, gleich oder verschieden substituiertes Phenyl oder Naphthyl steht, wobei als Substituenten insbesondere infrage kommen: Fluor, Chlor, Brom, Methyl, Ethyl, jeweils geradkettiges oder verzweigtes Propyl, Butyl, Pentyl, Hexyl, Heptyl, Octyl, Nonyl, Decyl oder Dodecyl, Methoxy, Ethoxy, jeweils geradkettiges oder verzweigtes Propyloxy, Butyloxy, Pentyloxy oder Hexyloxy, Methylthio, Ethylthio, jeweils geradkettiges oder verzweigtes Propylthio, Butylthio, Pentylthio oder Hexylthio, Trifluormethyl, Trichlormethyl, Trifluormethoxy, Trifluormethylthio, Trichlormethoxy, Trichlormethylthio, Propandiyl, Trimethylpropandiyl, Tetramethylpropandiyl, Dimethylpropandiyl, Triethylpropandiyl, Tetraethylpropandiyl, Butandiyl, Trimethylbutandiyl, Tetramethylbutandiyl, Tetraethylbutandiyl, jeweils gegebenenfalls ein- bis dreifach, gleich oder verschieden durch Chlor, Brom, Methyl oder Ethyl substituiertes Cyclopropyl, Cyclopentyl, Cyclohexyl, Cycloheptyl, Methoxycarbonyl, Ethoxycarbonyl, n- und i-Propoxycarbonyl, n- und i-Butoxycarbonyl, n- und i-Pentoxycarbonyl, n- und i-Hexyloxycarbonyl, Amino, N-Methylamino, N,N-Dimethylamino, N-Ethylamino, N,N-Diethylamino, N,N-Dipropylamino, N,N-Dibutylamino oder Phenyl,

$R^2$ für Methyl, Ethyl, n- und i-Propyl, n- und i-Butyl, n- und i-Pentyl, n- und i-Hexyl, n- und i-Heptyl, n- und i-Octyl, n- und i-Decyl, n- und i-Dodecyl, für Allyl, Butenyl, Pentenyl, Hexenyl, für jeweils gegebenenfalls ein- bis fünffach, gleich oder verschieden, durch Fluor, Chlor, Brom, Methyl oder Ethyl substituiertes Cyclopropylmethyl oder Cyclohexylmethyl oder für jeweils gegebenenfalls ein- bis dreifach, gleich oder verschieden durch Fluor, Chlor, Brom, Methyl, Methoxy, Ethyl, Ethoxy, n- und i-Propyl sowie n- und i-Propoxy substituiertes Benzyl oder Phenylethyl steht,

$R^3$ für Hydroxy, Methoxy, Ethoxy, n- und i-Propoxy, n- und i-Butoxy, n- und i-Pentoxy, n- und i-Hexyloxy, n- und i-Octyloxy, n- und i-Decyloxy, für jeweils gegebenenfalls ein- bis fünffach, gleich oder verschieden durch Fluor, Chlor, Brom, Methyl oder Ethyl substituiertes Cyclopropylmethoxy oder Cyclohexylmethoxy, für jeweils gegebenenfalls ein- bis dreifach, gleich oder verschieden durch Fluor, Chlor, Brom, Methyl, Methoxy, Ethyl, Ethoxy, n- und i-Propyl, sowie n- und i-Propoxy substituiertes Benzyl oder Phenylethyl, für Amino, N-Methylamino, N-Ethylamino, N-Propylamino, N-Butylamino, N-Pentylamino, N-Hexylamino, N-Octylamino, N,N-Dimethylamino, N,N-Diethylamino, N,N-Dipropyl-

amino, N,N-Dibutylamino, N,N-Dihexylamino, für gegebenenfalls ein- bis dreifach, gleich oder verschieden durch Fluor, Chlor, Brom, Methyl, Methoxy, Ethyl oder Ethoxy substituiertes N-Phenylamino oder für jeweils gegebenenfalls ein- bis dreifach, gleich oder verschieden durch Methyl, Ethyl und Isopropyl substituiertes 1-Piperidinyl, 1-Pyrrolidinyl, 4-Morpholinyl, 1-Perhydroazepinyl oder 1-Piperazinyl steht,

$R^4$ für Wasserstoff, Methyl, Ethyl, n- und i-Propyl, sowie n- und i-Butyl steht,

X für Schwefel oder die Sulfonylgruppe steht,

Y für Sauerstoff oder Schwefel steht, und

n für 0 oder 1 steht

wobei $R^1$ nur dann für unsubstituiertes Phenyl steht, wenn nicht gleichzeitig $R^2$ für Methyl, n für 1 und X für Schwefel stehen und wobei $R^1$ nur dann für 2-Nitrophenyl steht, wenn nicht gleichzeitig Y für Schwefel, n für 0 und $R^2$ für Methyl stehen.

4. Verfahren zur Herstellung von substituierten Pyrimidinen der allgemeinen Formel (I)

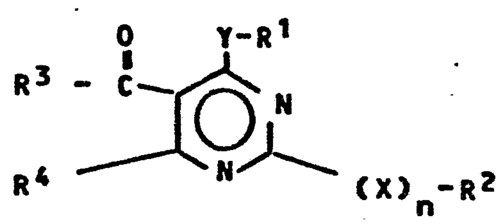

(I)

in welcher

$R^1$ für gegebenenfalls substituiertes Aryl steht,

$R^2$ für Alkyl, für Alkenyl, für gegebenenfalls substituiertes Cycloalkylalkyl oder für gegebenenfalls substituiertes Aralkyl steht,

$R^3$ für Hydroxy, für einen Rest $-O-R^5$ oder für

einen Rest 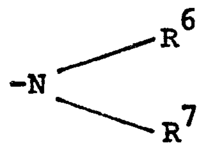 steht,

wobei $R^5$ für Alkyl, für gegebenenfalls substituiertes Cycloalkylalkyl oder für gegebenenfalls substituiertes Aralkyl steht und

$R^6$ und $R^7$ unabhängig voneinander für Wasserstoff, für Alkyl oder für gegebenenfalls substituiertes Aryl stehen oder

$R^6$ und $R^7$ gemeinsam mit dem Stickstoffatom, an welches sie gebunden sind, für einen gegebenenfalls substituierten gesättigten Heterocyclus stehen, der weitere Heteroatome enthalten kann,

$R^4$ für Wasserstoff oder Alkyl steht,

X für Schwefel oder die Sulfonylgruppe steht,

Y für Sauerstoff oder Schwefel steht,

n für 0 oder 1 steht,

wobei $R^1$ nur dann für unsubstituiertes Phenyl steht, wenn nicht gleichzeitig $R^2$ für Methyl, n für 1 und X für Schwefel stehen, und wobei $R^1$ nur dann für 2-Nitrophenyl steht, wenn nicht gleichzeitig Y für Schwefel, n für 0 und $R^2$ für Methyl stehen, sowie deren pflanzenverträglichen Säureadditionssalze, dadurch gekennzeichnet, daß man

(a) 4-Halogenpyimidine der Formel (II)

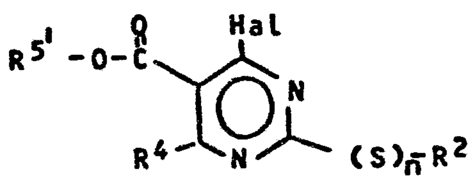

(II)

in welcher

Hal für Halogen steht,

$R^{5'}$ für Alkyl steht und

$R^2$,$R^4$ und n die oben angegebene Bedeutung haben,

mit aromatischen Alkoholen oder Thiolen der Formel (III)

$$R^1 - Y - H \qquad \text{(III)}$$

in welcher

$R^1$ und Y die oben angegebene Bedeutung haben,

gegebenenfalls in Gegenwart eines Verdünnungsmittels und gegebenenfalls in Gegenwart eines Säurebindemittels umsetzt zu den Verbindungen der Formel (Ia)

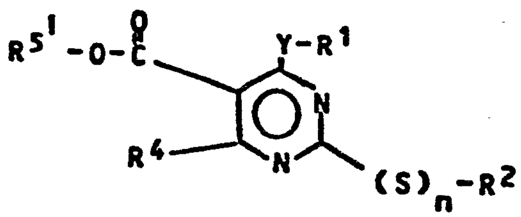

(Ia)

in welcher

$R^1$,$R^2$,$R^4$,$R^{5'}$,Y und n die oben angegebene Bedeutung haben,

oder daß man

(b) die nach Verfahren (a) erhaltenen 5-Alkoxycarbonyl-pyrimidine der Formel (Ia)

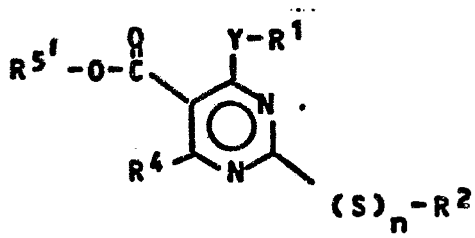

(Ia)

in welcher

$R^1$,$R^2$,$R^4$,$R^{5'}$,Y und n die oben angegebene Bedeutung haben,

mit Basen, gegebenenfalls in Gegenwart eines Verdünnungsmittels in üblicher Weise verseift zu den Pyrimidin-5-carbonsäuren der Formel (Ib)

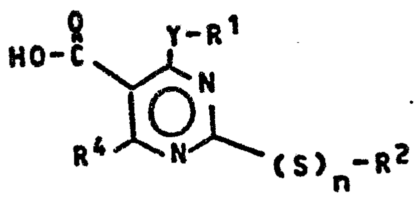

(Ib)

in welcher

$R^1$,$R^2$,$R^4$,Y und n die oben angegebene Bedeutung haben,

oder daß man

(c) die nach Verfahren (b) erhältlichen Pyrimidin-5-carbonsäuren der Formel (Ib)

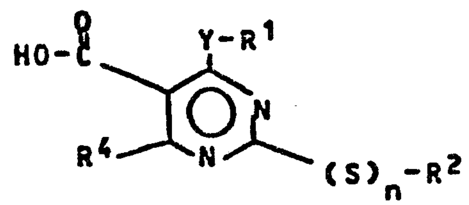

(Ib)

in welcher
$R^1$, $R^2$, $R^4$, Y und n die oben angegebene Bedeutung haben,

α) mit Alkoholen der Formel (IV),

$$R^5 - OH \qquad (IV)$$

in welcher

$R^5$ die oben angegebene Bedeutung hat,

gegebenenfalls in Gegenwart eines Verdünnungsmittels, und gegebenenfalls in Gegenwart eines Katalysators oder Aktivierungsmittels sowie gegebenenfalls in Gegenwart eines Säurebindemittels umsetzt zu den Pyrimidin-5-carbonsäureestern der Formel (Ic),

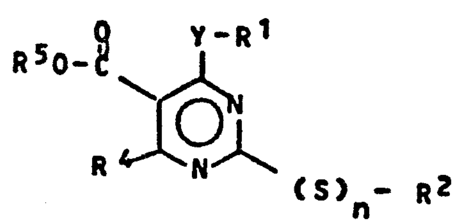

(Ic)

in welcher

$R^1, R^2, R^4, R^5$, Y und n die oben angegebene Bedeutung haben,

oder

(β) mit Aminen der Formel (V),

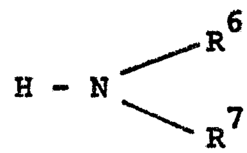

(V)

in welcher

$R^6$ und $R^7$ die oben angegebene Bedeutung haben,

gegebenenfalls in Gegenwart eines Verdünnungsmittels, und gegebenenfalls in Gegenwart eines Aktivierungsmittels sowie gegebenenfalls in Gegenwart eines Säurebindemittels umsetzt zu den Pyrimidin-5-carbonsäureamiden der Formel (Id),

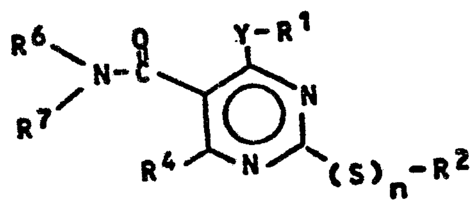

(Id)

in welcher

$R^1$,$R^2$,$R^4$,$R^6$,$R^7$,Y und n die oben angegebene Bedeutung haben,

oder daß man

(d) die nach Verfahren (a), (b), (c- ) oder (c-ß) erhältlichen Pyrimidin-Derivate der Formel

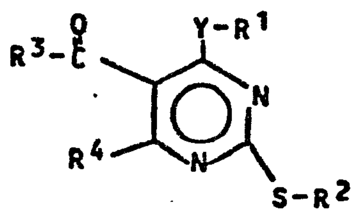

(Ie)

in welcher

$R^1$,$R^2$,$R^3$,$R^4$ und Y die oben angegebene Bedeutung haben,

mit Oxidationsmitteln der Formel (VI)

R - O - OH (VI)

in welcher

R für Wasserstoff oder für jeweils gegebenenfalls substituiertes Alkanoyl oder Aroyl steht,

gegebenenfalls in Gegenwart eines Verdünnungsmittels, und gegebenenfalls in Gegenwart eines Katalysators

sowie gegebenenfalls in Gegenwart eines Säurebindemittels oxidiert zu den 2-Sulfonylpyrimidinen der Formel (If)

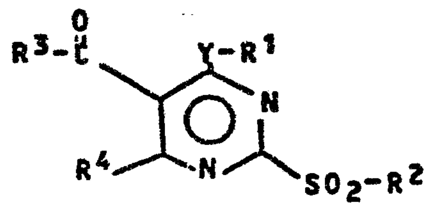

(If)

in welcher

$R^1$, $R^2$, $R^3$, $R^4$ und Y die oben angegebene Bedeutung haben,

und gegebenenfalls anschließend an die nach Verfahren (a), (b), (c-    ), (c-ß) oder (d) erhaltenen Pyrimidin-Derivate der Formel (I) eine Säure addiert.

5. Schädlingsbekämpfungsmittel, gekennzeichnet durch einen Gehalt an mindestens einem substituierten Pyrimidin der Formel (I) gemäß Ansprüchen 1 und 4.

6. Verwendung von substituierten Pyrimidinen der Formel (I) gemäß Ansprüchen 1 und 4 zur Bekämpfung von Schädlingen.

7. Verfahren zur Bekämpfung von Schädlingen, dadurch gekennzeichnet, daß man substituierte Pyrimidine der Formel (I) gemäß Ansprüchen 1 und 4 auf Schädlinge und/oder ihren Lebensraum einwirken läßt.

8. Verfahren zur Herstellung von Schädlingsbekämpfungsmitteln, dadurch gekennzeichnet, daß man substituierte Pyrimidine der Formel (I) gemäß Ansprüchen 1 und 4 mit Streckmitteln und/oder oberflächenaktiven Mitteln vermischt.